**Europäisches Patentamt**

(19) **European Patent Office**

**Office européen des brevets**

(11) Publication number : **0 478 550 B1**

(12) # EUROPEAN PATENT SPECIFICATION

(45) Date of publication of patent specification :
**25.01.95 Bulletin 95/04**

(51) Int. Cl.[6] : **G01N 27/327**, G01N 27/38,
C12Q 1/00, C12Q 1/40

(21) Application number : **90904262.4**

(22) Date of filing : **09.03.90**

(86) International application number :
**PCT/DK90/00067**

(87) International publication number :
**WO 90/10861 20.09.90 Gazette 90/22**

(54) **METHOD AND APPARATUS FOR DETERMINATION OF A CONSTITUENT IN A FLUID SAMPLE.**

(30) Priority : **09.03.89 DK 1159/89**

(43) Date of publication of application :
**08.04.92 Bulletin 92/15**

(45) Publication of the grant of the patent :
**25.01.95 Bulletin 95/04**

(84) Designated Contracting States :
**AT BE CH DE DK ES FR GB IT LI LU NL SE**

(56) References cited :
**EP-A- 0 078 636**
**EP-A- 0 127 958**
**WO-A-89/10395**
**GB-A- 1 223 363**
**US-A- 4 576 704**
**Analytical Chemistry, Vol. 62, 1990 J Wang et al: "Polishable and robust biological electrode surfaces", see page 318 - 320**

(73) Proprietor : **BRITISH TECHNOLOGY GROUP LIMITED**
**101 Newington Causeway**
**London SE1 6BU (GB)**

(72) Inventor : **COLIN, Fernand, Joseph, Guillaume**
**Avenue des Gerfauts 10**
**B-1170 Watermael-Boitsfort (BE)**

(74) Representative : **Chandler, Derek Richard et al**
**Patents Department**
**British Technology Group Ltd**
**101 Newington Causeway**
**London SE1 6BU (GB)**

EP 0 478 550 B1

## Description

### Field of the invention

The present invention relates to a method for measuring the concentration of at least one constituent of a fluid sample, e.g. glucose in a glucose-containing aqueous medium, for example a body fluid, in particular a whole blood sample, to an electrode device comprising the electrodes used in the measurement of the concentration of the constituent, to a sensor electrode for use in the method according to the invention and to an apparatus for use in the measurement of the concentration of the constituent in the sample, the apparatus preferably being portable.

### Description of prior art

Electrode devices and methods for detecting the amount of selected compounds in a liquid mixture are known in the art. Thus, in EP 78,636B and in EP 125,137A a sensor electrode for in vivo measurement of the amount of a component in a liquid mixture is described, wherein a charge-transfer mediator, such as ferrocene, is used as charge-transfer mediator in an enzyme-catalyzed reaction to transfer electrons arising from the reaction between the enzyme and a component in the liquid. The charge-transfer mediator ferrocene is adhered to the surface of the electrode in the form of a thin film layer or strip, for example by deposition from a solution. This known device suffers from severe drawbacks, i.e. the charge-transfer mediator will during the measurement rather rapidly be depleted from the strip causing a degradation of measurement reproducibility as explained into more details later. A similar device is described in EP 127,958A. The electrode devices are preferably intended for in vivo measurement of glucose in blood.

WO 89/10395 describes an enzyme electrode comprising a matrix made from a conductive powder, an enzyme and a charge-transfer mediator. The device suffers from the drawback that the concentration of the charge-transfer mediator is rather low. It is therefore necessary to operate the electrode at a rather high applied potential, leading to interference from other oxidizable substances in the sample.

The present invention provides an electrode device which eliminates the drawbacks of the prior art electrode devices.

### Summary of the invention

The present invention provides a method of voltametric detection of at least one constituent of a fluid sample of animal or human whole blood by means of a measuring apparatus containing an integrated electrode device including at least one enzyme sensor electrode and a reference electrode having an exposed surface part at a free end portion of the integrated electrode device and extending longitudinally through said end portion, said method being characterized in that there as reference electrode is used an Ag/AgCl electrode working at a chloride ion concentration of about 145 meq/l and that before applying the sample on the exposed surface of the electrode device, said electrode device is moved longitudinally outward by an increment and subsequently the outer end section of said free end portion of the integrated electrode device is removed by means of removing means mounted movably on the measuring apparatus, so as to provide a new exposed surface part of the integrated electrode device, and that subsequently said new exposed surface part of the integrated electrode device is exposed to said fluid sample, so as to generate a measuring signal representative of said constituent of the sample. When the exposed surface part of the measuring electrode or electrodes has been exposed to a first fluid sample for measuring a constituent thereof, part of the fluid sample will inevitably adhere to the exposed surface part. In order to avoid contamination of the next fluid sample to be measured it is necessary to secure that any residue of the first sample is efficiently removed. Furthermore, a fresh sensor electrode surface is also desirable to ensure reproducibility of measurements. This is obtained by the method according to the invention in which a new exposed surface part of the measuring electrode or electrodes is provided each time a measurement is to be made on a new fluid sample, namely by removing an outer end section of the free end portion of the electrode or electrodes.

From US 4 576 704 is known an apparatus for determining the oxygen content of a liquid. This apparatus has an electrode and insulating body which are via associated springs pressed to a grinding device, which serves to clean the electrodes. Further GB 1 223 363 describes tubular electrodes for the measurement of oxygen in a liquid. Deposits on these electrodes are removed via a grinding body which, upon rotation, cleans the front areas of the electrodes.

In a preferred embodiment, however, the free end portion of the electrode device is removed by cutting. Preferably, a thin slice is cut from the outer end portion of the electrode device, for example by means of cutting means formed like the blade of a plane.

It should be understood that the end surface of the electrode device formed by removing an outer end section form it may be convexly or concavely curved or may have a concave or convex conical or frusto-conical shape. In the latter case the removing means may operate like a pencil sharpener. In a preferred embodiment, however, the end surface of the electrode device including the exposed surface part of the electrode or electrodes is substantially plane.

The method according to the invention is especially useful in connection with the measurement of the content of glucose in blood.

Preferable is used an enzyme sensor electrode in the form of a paste comprising electrically conductive particles and a pasting material, containing an oxidase enzyme and a charge-transfer mediator being uniformly distributed in the paste, and in that the charge-transfer mediator in its reduced form is dissolved in the pasting material in a concentration which is at least sufficient to ensure that the concentration of the charge-transfer mediator in its oxidized form is substantially constant at the exposed surface of the sensor electrode during the measurement, the charge-transfer mediator in its reduced form optionally further being present in undissolved form in the paste.

The paste may be a paste comprising graphite particles and a non-polar pasting material.

The concentration of the reduced form of the charge-transfer mediator in the pasting material may be from 0.01 to 1.5 M, preferably from 0.05 to 1.0 M, more preferred from 0.07 to 0.7 M, and most preferred from 0.4 to 0.7 M, and as charge-transfer mediator an organo-metallic compound, the reduced form of which is soluble in a non-polar pasting material may be used. The organometallic compound may be a metallocene or a derivative thereof, preferably a ferrocene derivative, preferably 1,1'-dimethylferrocene. As non-polar pasting material paraffin oil may be used.

The measurement may be performed at a potential between the sensor electrode and the Ag/AgCl reference electrode in communication with the fluid sample in a range of from 0 to 250 mV. Preferred is a potential between the sensor electrode and the Ag/AgCl reference electrode in a range of from 0 to 200 mV, preferably from 10 to 150 mV, and in particular from 20 to 120 mV.

The measurement may be performed at a potential of about 110 mV, about 50mV or about 30 mV.

The method according to the invention may be used for measuring that the concentration of glucose in body fluids, such as whole blood, blood plasma, serum, urine or saliva, in particular in whole blood, can be determined by use of a reliable and reproducible _in vitro_ method according to the invention which method is based on a novel electrochemical principle using a charge-transfer mediator present in a non-polar medium in a sensor electrode which operates at a constant potential which is sufficiently low to substantially avoid interference resulting from oxidation of other oxidizable substances, such as acetylsalicylic acid (aspirin), paracetamol (acetaminophen), ascorbic acid, and sulfonylureas (tolbutamide, glibenclamide), present in the aqueous sample, such as a sample of a body fluid, in particular a whole blood sample.

In one aspect, the present invention relates to a method for determining glucose in a sample of an aqueous medium, the method being advantageous in that a substantially specific measure of the concentration of glucose is obtained without any significant uncertainty arising from a co-determination of interfering substances which, in general, can be present in the aqueous medium, in particular in blood. The method is based on monitoring the flow of current at an electrically conductive sensor electrode when a glucose oxidase catalyses a redox reaction of glucose in the presence of a charge-transfer mediator, preferably a ferrocene derivative, such as 1,1'-dimethylferrocene.

According to another aspect, the present invention provides an electrode device for use in the method described above, said electrode device comprising an electrode body member having a free end portion, at least one sensor electrode extending axially through said end portion and having a substantially uniform cross-sectional area within said end portion, and a reference electrode also extending axially through the free end portion of the electrode body member, each electrode being received in a bore extending longitudinally through the electrode body member and having an exposed surface part at the free end portion of the electrode body member, this electrode device being characterized in that the reference electrode is an Ag/AgCl electrode and that the sensor electrode is an electrically conductive electrode comprising a paste of electrically conductive particles and a pasting material containing an oxidase enzyme and a charge-transfer mediator, being uniformly distributed in the paste and in its reduced form being dissolved in the pasting material in a concentration which is at least sufficient to ensure that the concentration of the charge-transfer mediator in its oxidized form is substantially constant at the exposed surface of the sensor electrode during the measurement, the charge-transfer mediator in its reduced form optionally further being present in undissolved form in the paste. Because the cross-sectional area of each of the sensor or measuring electrodes positioned in the free end portion of the electrode body member is substantially uniform, the exposed active surface area of each electrode remains substantially unchanged when an outer end section or slice is removed from the free end portion.

The electrode device may further contain a counter electrode extending axially through the free portion of the electrode body member, the paste may comprise graphite particles and a non-polar pasting material which is substantially immiscible with water and which is capable of dissolving the charge-transfer mediator in its reduced form, and the oxidase enzyme may be glucose oxidase which is bonded and/or adsorbed to the electrically conductive particles.

The electrode device according to the invention may further be characterized in that the concentration of the reduced form of the charge-transfer mediator in the pasting material is from 0.01 to 1.5 M, preferably from 0.05 to 1.0 M, more preferably from 0.07 to 0.7 M, and most preferred from 0.4 to 0.7 M. The non-polar pasting material may be paraffin oil.

The electrode body may be made from a polymeric material. This material may be a mixture of high density polyethylene and low density polyethylene, the weight ratio between the amount of high density and low density polyethylene preferably being about 1.

The electrode device preferably comprises two sensor electrodes, an Ag/AgCl reference electrode and a counter electrode. The two sensor electrodes may be of the same type, but may be different, one electrode comprising glucose oxidase in a catalytically active form, the other electrode comprising glucose oxidase in an inactivated form.

The measurement is performed by bringing a surface of the electrode device, the surface suitably corresponding to a cross section of the electrode device, such as a perpendicular cross section, and comprising cross sections of all the electrodes present, into contact with a sample of the aqueous medium, the sample preferably being a sample of body fluid, in particular a drop of whole blood, which is taken from a patient, preferably by the patient himself, substantially immediately before the measurement is to be made. Before every measurement, the sensing surface of the electrode device is renewed by cutting off a thin slice of the electrode device in order to ensure the removal of any contaminants present on the sensing surface and to provide a fresh sensing surface of the electrode device. In addition, the removal of the sensor electrode sensing surface which has been exposed to the aqueous medium in question during the preceding measurement serves to ensure that the required amount of glucose oxidase and charge-transfer mediator is present at the surface of the electrode(s). Thus, a reliable and reproducible determination of the glucose concentration in a sample of an aqueous medium, such as a sample of body fluid, in particular a whole blood sample, without any interference present from contaminants from earlier measurements is obtained.

The present invention also provides a measuring apparatus for use in the method described above using an electrode device as described above, said apparatus comprising an apparatus body member defining an outwardly open cavity for receiving the electrode device therein, removing means mounted on the body member so as to be movable in relation thereto along a path intersecting said cavity for removing said outer end section from the free end portion of the electrode device received in the cavity, means for moving the electrode device outwardly by an increment each time the removing means has been operated an electronic measuring circuitry for processing measuring signals received from the electrode or electrodes of the electrode device, and connecting means for electrically connecting the electrode or electrodes of the electrode device to the electronic measuring circuitry when the electrode device is received in said cavity. The measuring apparatus may be made in pocket size so that it may be carried by a patient, such as a diabetic, who may use the apparatus for currently testing of the glucose content of his or hers blood. When an electrode device has been positioned in the cavity of the apparatus and the electrode of the electrode device has been connected to the electronic measuring circuitry of the apparatus, such electrode device may be used for measuring or testing a plurality of blood samples. When a test or measurement has been made, the removing means are operated so as to remove the outer end section from the free end portion of the electrode device. The apparatus preferably comprises means for moving the electrode device outwardly by an increment each time the removing means has been operated so that the exposed active surface part of each of the electrodes is always in substantially the same position in relation to the apparatus when a measurement is being made. As indicated above, the end surface of the electrode device may be concavely curved. Thus, the end surface of the electrode device may in itself and/or in combination with adjacent wall parts of the measuring apparatus define a cup-shaped container for receiving a liquid sample such as a drop of blood to be tested.

Preferably the removing means define at least one cutting edge for cutting the outer end section from the outer end portion of the electrode device when the removing means are moved along said path. The said cutting edge or edges may be rectilinear or convexly or concavely curved.

The removing means may comprise a plane or curved cutting blade mounted so as to be movable reciprocatingly along said path. Alternatively, the removing means may be mounted on the apparatus body member so as to be rotatable in relation thereto. When in the latter case the removing means comprises a cutting blade, the removing means may be formed like a pencil pointer or comprise a cutting knife functioning like that of a bread slicer. It should be understood that the removing means could also be of the non-mechanical type and could comprise means for cutting the outer end section from the free end portion of the electrode device by means of a laser beam or similar non-mechanical cutting means.

When the removing means are mounted rotatably in relation to the apparatus body member, the apparatus may further comprise means for permitting rotation of the removing means in one direction only, and such rotation permitting means may, for example, comprise a ratchet mechanism.

The moving means for incremental moving the electrode device outwardly in the cavity of the measuring apparatus each time the removing means have been operated may comprise a driving member driven by the movement of said removing means. Thus, the reciprocating or rotating movement of the removing means automatically causes that the electrode device is moved outwardly by an increment corresponding to the thickness of the end section or slice to be removed from the free end portion of the electrode device.

The removing means may be mounted on a lid-like member which is movable to a closing, non-operative position in which said exposed surface part of the measuring electrode or electrodes is covered and protected by the lid-like member, so that the active exposed electrode surface part is well protected when the measuring apparatus is carried, for example in a pocket or a hand bag.

When the apparatus is used for measuring a constituent, such a glucose, in the blood of a patient, such as a diabetic, the apparatus preferably further comprises a skin puncturing member mounted in the apparatus body member so as to be movable between retracted and extended positions, biassing means for biassing the puncturing member towards its extended position, and releasable locking means for retaining the puncturing member in its retracted position against the bias of the biassing means, whereby the puncturing member may perform a sudden skin puncturing movement from its retracted to its extended position, when the locking means are released. The apparatus may comprise a skin contacting surface, and the puncturing member may be arranged totally within the apparatus body member or housing in its retracted position, while the puncturing member may protrude from the skin contacting surface of the apparatus in its extended position. When the apparatus is to be used, the patient may place the tip of his finger or another skin surface part in contact with the skin contacting surface of the apparatus, whereafter the locking means may be released. When the skin of the patient has been punctured, the patient may apply a drop of blood to the exposed end surface of the electrode device.

The apparatus may also comprise a visual display for displaying the result of the measurement performed by the electrode or electrodes of the electrode device, so that the result or results of each measurement may be directly read from the display.

The apparatus according to the invention can preferably be used for determining the concentration of glucose in an aqueous medium, the apparatus comprising an electrode device containing a sensor electrode, the apparatus preferably being portable and well suited for use by patients suffering from diabetes in order to self-monitor the glucose level in a body fluid, in particular in whole blood. Furthermore, certain embodiments of the apparatus of the invention are particularly useful as a diagnostic tool in hospital wards for intensive care or therapy, or in internal or general medicine as well as in veterinary medicine.

**Detailed disclosure of the invention**

**Description of the principle of the preferred method according to the invention**

The basis of the preferred method according to the invention for measuring the concentration of glucose in a sample of a glucose-containing aqueous medium, which can be performed by means of an apparatus comprising an electrode device according to the present invention, is the reaction between glucose and the enzyme glucose oxidase (GOD). Electrons produced by this oxidation reaction are transferred to a charge-transfer mediator, e.g. a metallocene or a derivative thereof, and finally captured by a conductive material in the sensor electrode, e.g. graphite particles. The latter enzyme-catalyzed oxidation of glucose leads to gluconic acid and the "reduced form" of glucose oxidase, i.e. glucose oxidase wherein at least one functional group has been reduced. This reduced enzyme reacts with the oxidized form of the charge-transfer mediator, which is formed by donation of an electron from the "reduced" form of the charge-transfer mediator to the electrically conductive sensor electrode, and the glucose oxidase is thereby regenerated together with the reduced form of the charge-transfer mediator. The electrical current produced in the process is proportional to the concentration of glucose present in the sample.

The reactions can schematically be described by the following equations:

$$\text{Glucose} + \text{glucose oxidase-FAD} + H_2O \rightarrow \text{gluconic acid} + \text{glucose oxidase-FADH}_2 \quad \text{I}$$

$$\text{glucose oxidase-FADH}_2 + 2\,CTM_{ox}^+ \rightarrow \text{glucose oxidase-FAD} + 2\,CTM_{red} + 2H^+ \quad \text{II}$$

$$2\,CTM_{red} \rightarrow 2\,CTM_{ox}^+ + 2e^- \quad \text{III}$$

where FAD designates the oxidized form of the flavin-adenine dinucleotide part of glucose oxidase, $FADH_2$ designates the reduced form of the flavin-adenine dinucleotide part of glucose oxidase, $CTM_{ox}$ designates the

charge-transfer mediator in oxidized form and $CTM_{red}$ is the charge-transfer mediator in reduced form.

A simplified way of expressing the reactions is:

$$glucose + GOD \rightarrow gluconic\ acid + GOD^- \quad IV$$
$$GOD^- + CTM_{ox}^+ \rightarrow GOD + CTM_{red} \quad V$$
$$CTM_{red} \rightarrow CTM_{ox}^+ + e^- \quad VI$$

The two first reactions (I,II or IV,V, respectively) take place at the surface of the liquid of the sample and the exposed surface of the sensor electrode, and the oxidation reaction of the CTM (III or VI, respectively) takes place in the electrode.

The above formalism refers to the use of a charge-transfer mediator which undergoes a one-electron redox process. However, charge-transfer mediators which undergo, e.g. a two-electron redox process may also be useful within the context of the present invention.

In theory, the system is a system wherein the glucose oxidase and the charge-transfer mediator are consumed in the process, followed by a regeneration of the parent substances in the same amounts as consumed. In principle, this should lead to a cyclically functioning system with a long period of function.

Electrodes based on the above general principle are known, but they suffer from problems caused by the depletion of the charge-transfer mediator, due to relatively high water-solubility of the oxidized form thereof.

A significant extent of depletion of the charge-transfer mediator can result in several drawbacks, e.g.

(i) only one measurement can be performed in a given sample owing to rapid loss of the charge-transfer mediator to the medium of the sample;

(ii) the reproducibility of the measurement is poor as the extent of the depletion of the charge-transfer mediator is rather unpredictable and depends on the time elapsing between exposure of the sample to the electrodes in question and the performance of the actual measurement;

(iii) the sensitivity at low glucose concentrations is very poor; and

(iv) the applied potential between the sensor electrode and the reference electrode has to be rather high, leading to interference from other oxidizable substances present in the aqueous sample.

A prerequisite for a successful and reproducible measurement of the specific glucose concentration in a body fluid, in particular in whole blood, based on the above-mentioned principle using a suitable apparatus comprising the sensor electrode, a suitable reference electrode and a suitable counter electrode is thus that a sufficiently well-defined amount of the charge-transfer mediator is present throughout the measurements. Furthermore, it is advantageous if the measurement can be repeated without taking a new sample of blood and without replacing the sensor electrode.

The present invention provides such a method based on the above-mentioned electrochemical principle for measuring the glucose concentration in a sample of an aqueous medium, e.g. a sample of body fluid, such as a sample of whole blood, blood plasma, serum, urine or saliva, but the sensor electrode(s) has/have a composition and a structure which ensure that the charge-transfer mediator is present and available in an amount sufficient to ensure that the concentration of its oxidized form is maintained substantially constant during the measurement, whereby the sensor electrode(s) work(s) at a low potential in the range of 0-250 mV relative to a silver/silver chloride reference electrode operating at about 145 meq/liter chloride ion concentration (the latter concentration being of the same order as that in human plasma (about 100 meq/l)). Furthermore, the measurement can be repeated with the same sample and electrode device without any further discomfort to the patient.

It has been found that operating the sensor electrode at a relatively low potential results in a better signal-to-noise ratio and a better accuracy and reproducibility of the measurements.

Furthermore, operation of the sensor electrode at a relatively low and constant potential is very important if interference from oxidizable substances other than glucose present in the blood is to be avoided. This is explained in the following, noticing that the expression "redox potential" is intended to mean the redox potential relative to a reference electrode which is comparable to the reference electrode used according to the present invention.

Substances which have redox potentials which are lower than the redox potential of the charge-transfer mediator, e.g. 1,1'-dimethylferrocene, at which the sensor electrode is working are, if present in the sample, co-determined by the method according to the invention. To our knowledge only very few, if any, oxidizable substances which have a redox potential below the potential of 1,1'-dimethylferrocene may in general, be present in a body fluid, in particular in blood, from subjects, e.g. patients, mainly suffering from diabetes mellitus. The method of the invention has the advantage that the potential at which the electrode device operates can be kept relatively low, since the oxidation of glucose to gluconic acid is achieved enzymatically rather than electrolytically, and the reduced form of the charge-transfer mediator is present in such large amounts that (a) the percentage depletion of the reduced form due to loss of the oxidized form to the aqueous medium is very small, so that the concentration of the reduced form within the sensor electrode may be regarded as being essentially constant, and (b) the applied potential required to bring about the redox process for the CTM/CTM$^+$ is relatively low.

One way of eliminating the contribution resulting from any possible interfering substance is to use a technique which enables the contribution from any interfering substance to be subtracted from the total signal in order to give a reliable measurement of the glucose concentration.

The preferred method according to the present invention is a method which is performed at a relatively low and substantially constant potential and at the same time is reproducible and has a high sensitivity . Furthermore, the method according to the invention enables the use of two different sensor electrodes (i.e. a blank sensor electrode comprising inactivated glucose oxidase (e.g. denatured by heating) and a measuring sensor electrode comprising active glucose oxidase), whereby signals resulting from interfering substances present in the aqueous sample are monitored via the blank sensor electrode and signals corresponding to the total concentration of glucose and interfering substances present in the aqueous sample are monitored via the sensor electrode comprising the active glucose oxidase. Thus, by subtracting the former signal from the latter a specific measure of the concentration of glucose in the sample can be obtained.

Until now, no such sensitive and substantially specific method for measuring the concentration of glucose in a body fluid, in particular in whole blood, has been described.

## The electrode device

The electrode device according to the present invention comprises an electrode device body (also denoted an electrode body member) and the electrodes. In the following, the construction of preferred embodiments of the electrode device is described.

### The electrode device body

The electrode device body may have a cylindrical rod-like shape. It is made of a sliceable polymeric material. If necessary, additives may be included to obtain a polymeric material which has a sufficient hydrophilicity to ensure favorable contact between the aqueous sample and the electrode device.

The electrode body member is suitably made from a plastic material, such as a polymeric material. Such material may be of an electrically insulating or electrically conductive type. In the latter case the electrode body member may in itself function as an electrode as indicated above.

The electrode device body is suitably made of polymeric materials such as polyethylene(s), EVA, polyurethane, or polyvinylchloride(s). A preferred material is polyethylene, such as high density polyethylene or low density polyethylene, especially in admixture, the ratio between the high density and the low density polyethylene then being in the range of between 1:10 and 1:0.1, preferably in the range of between 1:5 and 1:0.5, in particular about 1:1 by weight. Suitable additives can be used to give better wetting of the surface of the electrode device, i.e. establish a better contact between the sample of the aqueous medium and the surface of the electrode device. Suitable additives are polyethylene oxide, glycerol fatty acid esters or N,N-bis(2-hydroxyethyl)dodecanamide.

The electrode device body contains at least two longitudinal channels wherein each of the electrodes are positioned. In the case of two channels, the electrodes which are placed in the channels are a sensor electrode and a combined reference and counter electrode. In the case of three channels, the electrodes are a sensor electrode, a reference electrode and a counter electrode, respectively, or two sensor electrodes (which are the same or different) and a combined reference and counter electrode. In a preferred embodiment, the electrode device body contains four channels, two of which house a sensor electrode, the sensor electrodes being the same or different, and the other two housing a reference and a counter electrode, respectively.

The longitudinal the channel(s) housing the sensor electrode(s), has/have substantially the same cross-sectional diameter throughout the length of the channel in order to ensure that the exposed sensing surfaces of all the electrodes remain constant from one measurement to the next. Thus, the removal of a thin slice of the electrode device before a new measurement, resulting in the exposure of a fresh surface, does not result in a change of the area(s) of the exposed surface(s) of the sensor electrode(s) included in the electrode device body. This characteristic is very important, since a variation in the area(s) of the exposed surface(s) of the sensor electrode(s) from one measurement to another leads to a corresponding, approximately linear variation in the current signal, and thus gives rise to a completely new measuring condition leading to another calibration of the system, i.e. the linear correlation between the concentration of glucose in the sample and the current generated at the sensor electrode changes its parameters (i.e. slope and intercept). By keeping the area(s) of the exposed surface(s) of the sensor electrode(s) substantially constant, i.e. the diameter of each channel is substantially constant throughout its length, the correlation between glucose and current signal is maintained substantially constant.

*Electrodes*

Preferably, the electrode device according to the present invention comprises a sensor electrode, a reference electrode and a counter electrode. In a preferred embodiment two sensor electrodes are present.

Sensor electrode

The preferred embodiment of a sensor electrode of the invention comprises an electrically conductive material, together with glucose oxidase and the charge-transfer mediator.

The electrically conductive material is preferably a carbon-based material such as surface-oxidized graphite particles. The size of the particles should be at most 50 μm, preferably 1-20 μm.

In a preferred embodiment, the electrically conductive material is a carbon-based paste containing a pasting material. Useful pasting materials are non-polar substances which are substantially immiscible with water, such as paraffin, paraffin oil, silicone oil etc., paraffin oil being preferred.

The amount of the non-polar pasting material present in the paste is suitably about 30-40%, preferably about 30-45% given as weight percentages based on the total volumes of carbon-based, e.g. graphite-based, particles and the pasting material.

The pasting material constitutes a support and a dispersion medium for a charge-transfer mediator and regulates the release of the charge-transfer mediator in a controlled way during the measurement. Furthermore, the pasting material serves as a cohesive medium by filling the interstices between the graphite particles.

The charge-transfer mediator which is present in the sensor electrode is preferably an organometallic compound having a redox potential $E_0$ in the range of 0-150 mV, the organometallic compound preferably being very soluble in a non-polar pasting material, such as paraffin oil, and substantially insoluble in water. A preferred type of organometallic compound is a metallocene derivative, the reduced form of which is soluble in the non-polar pasting material. The metallocene derivative may suitably be a ferrocene derivative, i.e. a derivative of bis(cyclopentadienyl)iron(II), but other metallocenes or metallocene derivatives containing, e.g., nickel (as Ni(II)) or ruthenium (as Ru(II)) may also be used.

Preferred charge-transfer mediators for use according to the present invention are ferrocene derivatives, in particular 1,1'-dimethylferrocene.

Within the sensor electrode, the charge-transfer mediator is present in the reduced form (e.g. as 1,1'-dimethylferrocene). Due to the relatively non-polar nature of the charge-transfer mediator in reduced form and its low solubility in aqueous medium it is predominantly present in the non-polar pasting material, the pasting material thus functioning as high-capacity reservoir of charge-transfer mediator.

In addition, the charge-transfer mediator may, depending on its solubility in the non-polar pasting material, also be present in the form of solid particles.

In a preferred embodiment the pasting material is paraffin oil and the charge-transfer mediator is 1,1'-dimethylferrocene, the latter being present in the paraffin oil in a concentration of about 0.01 to about 1.5 M, preferably about 0.05-1.0 M, in particular 0.07-0.7 M, most preferred about 0.4-0.7 M.

The amount of charge-transfer mediator in relation to the graphite particles is, in the case of 1,1'-dimethylferrocene as the charge-transfer mediator, in the range of 1-350 mg 1,1'-dimethylferrocene per gram GOD-containing graphite, preferably 1-100 mg 1,1'-dimethylferrocene per gram GOD-containing graphite, in particular 10-75 mg 1,1'-dimethylferrocene per gram GOD-containing graphite.

In one preferred embodiment the concentration of 1,1'-dimethylferrocene in the paraffin oil is about 0.1 M, and 12.85 mg 1,1'-dimethylferrocene is present per gram GOD-containing graphite.

In another preferred embodiment the concentration of 1,1'-dimethylferrocene in the paraffin oil is about 0.5 M, and 64.25 mg 1,1'-dimethylferrocene is present per gram GOD-containing graphite.

The enzyme preferred in the method according to the invention is glucose oxidase, which is a oxidoreductase enzyme of the classification EC 1.3.4. However, other enzymes which are oxidoreductases may be used, e.g. glucose dehydrogenase, L-amino acid oxidase or a glycolate oxidase.

The glucose oxidase preferred as enzyme in the sensor electrode is attached to the surface oxidized graphite particles and/or immobilized on the latter particles by means of a treatment involving the use of a carbodiimide reagent such as, e.g., 1-cyclohexyl-3-(2-morpholino-ethyl)carbodiimide metho-p-toluenesulfonate (N-cyclohexyl-N'-β-[(N-methylmorpholino)ethyl]carbodiimide p-toluenesulfonate salt). The glucose oxidase is present in the sensor electrode in an amount of at least 500 IU per gram of graphite, preferably in an amount of at least about 1000 IU, such as at least about 2000 IU, more preferably at least about 4000 IU, most preferably at least about 6000 IU per gram of graphite, especially about 8000 IU per gram graphite.

An essential feature of the sensor electrode is that the glucose oxidase and the charge-transfer mediator are substantially uniformly or homogeneously distributed in the carbon-containing paste. In this way it is possible to cut off a thin, cross-sectional slice of the electrode and at the same time ensure that the correct and sufficient amount of glucose oxidase and charge-transfer mediator is present at the working surface of the electrode, the working surface of the sensor electrode being the surface which is exposed to the glucose-containing aqueous medium, such as a blood sample.

In a preferred embodiment of the sensor electrode for determination of glucose, two sensor electrodes are present in the electrode device. The two sensor electrodes can be similar or different. When the sensor electrodes are different, one electrode comprises the glucose oxidase in an active form and the other comprises the glucose oxidase in an inactive form which has been inactivated by heat denaturation at 50°C for 24 hours.

The advantage of using two similar sensor electrodes for the measurements is that it is possible in this way to obtain two responses, one from each sensor electrode. The responses should be substantially equal if the measurement is performed correctly. Different responses from two similar electrodes is an indication of an unreliable measurement due e.g. to inadequate contact between the sample and the electrodes.

The advantage of using two different sensor electrodes, one containing the active enzyme and the other one containing the inactivated enzyme, is that it is possible in this way to obtain a blank response from the electrode containing the inactivated enzyme, whereby response from interfering substances can be subtracted from the measurement performed at the sensor electrode containing the active glucose oxidase.

In the method according to the invention, the measurement of the concentration of glucose in a sample of an aqueous medium is performed at a potential between the sensor electrode and a reference electrode in communication with the sample of the aqueous medium which is in a range which is about 0-250 mV when the reference electrode is an Ag/AgCl reference electrode working at a concentration of chloride ions of about 145 meq/l, or, when the reference electrode is different therefrom, which is in a range corresponding a range of about 0-250 mV measured versus an Ag/AgCl reference electrode working at a concentration of chloride ions of about 145 meq/l.

If, e.g., the applied potential at the sensor electrode versus a reference electrode comprising Ag/AgCl in a 1 M solution of KCl ($[Cl^-]_a$) is 160 mV, this value corresponds to an applied potential of 110 mV, when the measurement is performed with an Ag/AgCl electrode working at a concentration of chloride ions of about 145 meq/l ($[Cl^-]_b$). This relationship can be derived from the following equation corresponding to the Nernstian relationship:

$$E = E_o - (RT/nF) \ln ([Cl^-]_a/[Cl^-]_b)$$

By insertion of the relevant values in the equation it can be found that

$$E = E_o - 50 \text{ mV}$$

showing that a change in the reference electrode from a reference electrode comprising Ag/AgCl in a 1 M solution of KCl in an agarose gel to a reference electrode comprising Ag/AgCl working at a concentration of chloride ions of 145 meq/l leads to a shift in the potential from 160 mV to 110 mV.

In preferred embodiment of the method according to the invention the potential between the sensor electrode and the Ag/AgCl reference electrode working at a concentration of chloride ions of about 145 meq/l is in the range of about 0-200 mV, preferably in the range of 10-150 mV, in particular in the range of 20-120 mV, such as 30 mV, 50 mV or 110 mV.

*Preparation of the sensor electrode*

A preferred sensor electrode for the determination of glucose may be prepared in the following manner in which the preparation of the electrode material as well as the filling of the channel(s) of the sensor electrode in the electrode device body is performed:

The carbon particles, preferably graphite particles, are surface oxidized by heating, e.g. at 100°C for a predetermined period in a well ventilated, continuously rotated vessel in the presence of dry atmospheric air. These surface oxidized graphite particles are activated with 1-cyclohexyl-3-(2-morpholinoethyl)carbodiimide metho-p-toluenesulfonate (N-cyclohexyl-N′-[β-(N-(methylmorpholino)ethyl]carbodiimide p-toluenesulfonate salt), e.g. 42.3 mg per gram surface oxidized graphite particles. The activation is performed by mixing the graphite particles with the 1-cyclohexyl-3-(2-morpholinoethyl)carbodiimide metho-p-toluenesulfonate dissolved in an aqueous buffer solution, e.g. an acetate buffer of pH 4.76, followed by continuous stirring for e.g., about 2-4 hours at room temperature. After stirring, the mixture is washed several times (e.g. 4-7 times) with distilled water until approximately neutral pH (5-7), the carbodiimide-activated graphite particles are dried by evaporation of the water, e.g. by forced evaporation using a fan, or under reduced pressure, at room temperature or by lyophilization. Before immobilization of the glucose oxidase on the carbodiimide-activated graphite, the glucose oxidase is dissolved in an aqueous buffer having a suitable pH at which the enzyme is sufficiently stable, and containing a coupling reagent for coupling the enzyme to the carbodiimide-activated graphite, e.g. a phosphate buffer having a pH of 7.3 and containing 4 % w/w glutaraldehyde (i.e glutaric dialdehyde). The amount of buffer used is, e.g., 2 ml per gram carbodiimide-activated graphite particles and the amount of enzyme used is, e.g., at least 4000 IU, preferably 8000 IU per gram of the carbodiimide-activated graphite particles. The immobilization is performed by suspending the carbodiimide-activated graphite particles in the buffer containing the enzyme and the coupling reagent. The suspension is stirred continuously for about 16 hours at 4°C followed by removal of the water by evaporation, e.g. at reduced pressure, at room temperature or by lyophilization. Using glutaraldehyde as coupling reagent the enzyme, preferably glucose oxidase, becomes covalently bonded to the graphite particles. However, physical adsorption of the enzyme to the surface of the particles may also be of some importance in relation to the immobilization of the enzyme on the particles.

In the following the term "GOD-containing graphite" is used to mean carbodiimide-activated graphite particles comprising the glucose oxidase, the particles being prepared in the manner described above.

The GOD-containing graphite is sieved through a 48 mesh (mesh size corresponding to a sieve having an opening of 297 μm) and the sieved material is then mixed with a sufficient amount of the non-polar pasting material, i.e. paraffin oil, in which the charge-transfer mediator, i.e. 1,1'-dimethylferrocene, has been dissolved. Preferably, the concentration of the 1,1'-dimethylferrocene solution is about 0.01-1.5 M, preferably about 0.05-1.0, in particular about 0.07-0.7 M, in particular about 0.1-0.7 M, most preferred about 0.4-0.7 M in paraffin oil. The amount of the 1,1'-dimethylferrocene in paraffin oil is larger than the required and final amount in the sensor electrode. The reason for this is that it should be possible to obtain a dispersion of GOD-containing graphite in the 1,1'-dimethylferrocene solution, the dispersion having a consistency which allows the appropriate channel(s) in the electrode device body to be filled. The excess amount of the 1,1'-dimethylferrocene solution is easily removed after filling of the channel(s) of the sensor electrode in the electrode device body by centrifugation. The amount of 1,1'-dimethylferrocene used is preferably 3-4 times greater than the amount necessary to establish a final concentration of about 10-75 mg 1,1'-dimethylferrocene per gram GOD-containing graphite in the sensor electrode, and an amount of about 2-3 ml 1,1'-dimethylferrocene solution, preferably with a concentration of about 0.07-0.7 M, more preferred in a concentration of about 0.4-0.7 M, is normally used per gram GOD-containing graphite.

To fill the sensor electrode channels(s), the electrode device body is placed in a special holder having a small funnel-shaped reservoir at the top. The dispersion containing the GOD-containing graphite and the 1,1'-dimethylferrocene in paraffin oil solution is then poured into the reservoir. The electrode device body equipped with the filled reservoir is then centrifuged at about 15,000 - 50,000 x g for about 5-30 min, the centrifugation preferably being performed at about 20,000 x g for about 10 min, the electrode body device being positioned in an inverted position during centrifugation (i.e. that end of the device from which slices later are removed being uppermost, the funnel-shaped reservoir being inserted in that end). By the centrifugation the air is displaced from the channels and the GOD-containing graphite particles sediment, the interstices between the particles thus being filled with the 1,1'-dimethylferrocene in paraffin oil, and the excess amount of 1,1'-dimethylferrocene in paraffin oil remaining as a supernatant in the upper part. After centrifugation the excess 1,1'-dimethylferrocene in paraffin oil is easily removed and the surface of the sensor electrode is levelled with the surface of the electrode device body by cutting off a suitable cross-sectional portion of the electrode device.

The advantages achieved by using the above-mentioned method of preparing and filling the sensor electrode channel(s) in the electrode device body is that the components of the sensor electrode(s) become packed uniformly or homogeneously in the graphite-containing paste, the interstitial spaces between the GOD-containing particles being filled with the 1,1'-dimethylferrocene in paraffin oil such that substantially no air bubbles remain within the sensor electrode(s).

Another way of filling the sensor electrode channel(s) in the electrode device body is by suitable fixation of the electrode device body and subsequent filling in vacuo; for example the sensor electrode channel(s) can be filled by compression of the GOD-containing paste into the channel(s) by use of a suitable equipment and punches to suit.

Another way of preparing the sensor electrode is to mix the GOD-containing graphite particles with a solution containing the charge-transfer mediator, preferably 1,1'-dimethylferrocene, dissolved in a mixture comprising n-pentane and the paste-forming material, e.g. paraffin oil. After intimate mixing the n-pentane is removed by evaporation and the paste is introduced into the channels in vacuo. n-Pentane can be replaced by other volatile, non-reacting organic solvents in which the charge-transfer mediator is soluble, e.g. petroleum ether.

Suitable amounts for preparation by the latter method are, e.g., about 10-15 mg 1,1'-dimethylferrocene, preferably 12.85 mg 1,1'-dimethylferrocene, 0.2-1.0 ml paraffin oil, preferably 0.6 ml paraffin oil, and 4-5 ml n-pentane per gram GOD-containing graphite.

Alternatively, suitable amounts are, e.g., about 50-75 mg 1,1'-dimethylferrocene, preferably 64.25 mg 1,1'-dimethylferrocene, 0.2-1.2 ml paraffin oil, preferably 0.6 ml paraffin oil, and 4-5 ml n-pentane per gram GOD-containing graphite.

## Reference electrode

The reference electrode can be any physically suitable reference electrode of a conventional type. E.g., it can be a silver/silver chloride reference electrode or a variant thereof comprising an aqueous solution of potassium chloride (e.g. a 1 M solution) in a gel (e.g. an agarose gel) and at the bottom having a silver/silver chloride electrode. In the method according to the present invention a silver/silver chloride electrode is preferred, the electrode comprising a silver wire on which a layer of silver chloride has been established by immersion in ferric chloride, or by an electrolytic treatment in 0.1 M hydrochloric acid. The silver wire is rather thin, preferably having a diameter of about 100-300 $\mu$m, in particular about 200 $\mu$m. Alternatively, the reference electrode can comprise an appropriate number of silver chloride coated silver wires, each wire having a diameter of about 20-100 $\mu$m, preferably about 30 $\mu$m.

Following removal of a cross-sectional slice of the electrode device prior to performing a measurement it is clear that the freshly exposed cross-sectional surface of the silver wire is no longer covered by silver chloride. However, upon exposure to a sample of an aqueous medium which contains at least a moderate concentration of chloride ions (such as whole blood or urine) the latter cross-sectional surface rapidly becomes coated with an adequate layer of silver chloride by reaction between the silver from the silver wire and the chloride present in the aqueous sample. The chloride present in the aqueous sample may arise from dissolution of silver chloride from the periphery of the exposed silver surface of the reference electrode itself and/or from the content of chloride ion in the sample. In the case where the aqueous sample itself contains only a small concentration of chloride ions it is thus advantageous to use a reference electrode comprising a number of silver wires, whereby a greater surface area of the silver chloride from the periphery of the reference electrode surface is available to release chloride ions to the aqueous sample, resulting in the formation of the necessary layer of silver chloride on the cross-sectional surface of the silver wire.

Alternatively, the reference electrode may be made of any sliceable plastic material in which is embedded a powder comprising small silver and silver chloride particles.

The reference electrode is suitably prepared by inserting the silver wire(s) on which the silver chloride layer has been established into the reference electrode channel of the electrode device body . The silver/silver chloride electrode is then fixed into position in the channel by injecting, e.g., two-component epoxy adhesive into the free space in the channel.

## Counter electrode

The counter electrode is preferably a silver wire of diameter about 100-300 $\mu$m, preferably about 200 $\mu$m (comparable to the wire used in a preferred embodiment of the reference electrode). It may also be made of any suitably electrically conductive sliceable polymer-based material, e.g. as a plastic material, or a electrically conductive carbon-based paste, e.g. a graphite-based paste.

During the measurement, the counter electrode delivers an electrical current which counterbalances the current generated at the sensor electrode as a result of the electrochemical reaction at the surface exposed to the sample, and as a result a fixed, constant potential between the sensor electrode and the reference electrode can be maintained.

## Theory

Operation of the sensor electrode of an electrode device according to the invention at relatively low and constant potential is very important if interference from oxidizable substances other than glucose present in the blood is to be avoided.

In addition, the measurement must be reproducible and accuracy should be high, particularly at low glucose concentrations (as would be the case, e.g, for a patient in hypoglycemic coma). The patient himself should be able to check the correct functioning of the electrode device by using a single test solution and finally it should be possible to manufacture the sensor on a large scale to strict specifications.

To understand how these conditions are fulfilled in the context of the invention, some understanding of the mechanism of operation of the electrode device of the invention, particular the sensor electrode, is necessary.

Without being limited to any specific theory, a model describing the principle involved is presented in the following:

The main points can be summarized as follows:

- At the interface between the pasting material (PM) and an aqueous medium, the ratio between the concentration of the charge-transfer mediator in the pasting material and that in the aqueous medium is determined by the appropriate partition coefficient. Thus by fixing the concentration in the pasting material, the concentration in the aqueous medium is thereby well-defined at the interface.

- The reduced form of the charge-transfer mediator (CTM) diffuses from the pasting material into the reaction layer in the immediate proximity of the cross-sectional surface of the sensor electrode. The flux is proportional to the gradient between the surface of the pasting material (PM) and the carbon (graphite) surface to which GOD is bound or adsorbed. The relation between the flux (F) and the gradient has the following form:

$$F = P (M_p - M_c) \quad (1)$$

$P$: apparent permeability ($cm.sec^{-1}$);

$M_p$: concentration of the mediator at the PM surface, assumed constant;

$M_c$: concentration of the mediator at the carbon surface.

At the carbon (graphite) surface in the sensor electrode, CTM is oxidized to $CTM^+$. As this may be presumed to be a Nernstian exchange, the following relation should approximately apply:

$$E - E_o = RT/nF \log ([CTM^+]/[CTM]) \quad (2)$$

$(n = 1)$

(where E is the electrode potential relative to a chosen reference point and $E_o$ is the standard electrode potential relative to the same reference point)

i.e.

$$[CTM^+]/[CTM] = \exp [ (E - E_o)F/RT ].$$

It should be recognized that the value of the potential, E, is a function of the ratio of $[CTM^+]$ to $[CTM]$ and not of the absolute concentration ($[CTM^+]$) of $CTM^+$.

Both species CTM and $CTM^+$ diffuse in the aqueous medium in the proximity of the sensor electrode surface in such a way that their actual concentrations in the reaction layer cannot be safely predicted.

Diffusion of both species must be compensated by the flux F. This must not exhaust the store of mediator contained in PM, and for this reason the charge-transfer mediator must be much more soluble in PM than in the aqueous medium. In the absence of glucose, the continuous production of $CTM^+$ at the graphite surface and its leakage into the surrounding aqueous medium (bulk) is responsible for part of the background diffusional current $I_d$. The remaining part of the background current is mainly due to the presence of undetermined extraneous chemicals in blood and even in GOD itself. Diffusion of the species CTM does not produce any current.

When the applied potential is progressively increased, starting from zero, the amount of $CTM^+$ increases correspondingly at the sensor electrode surface, increasing the leakage of $CTM^+$ and thus $I_d$. As the flux F increases, the concentration gradient between the PM surface and the carbon surface builds up and $M_c$ falls, and this gradient has a limiting value when $M_c$ falls to zero.

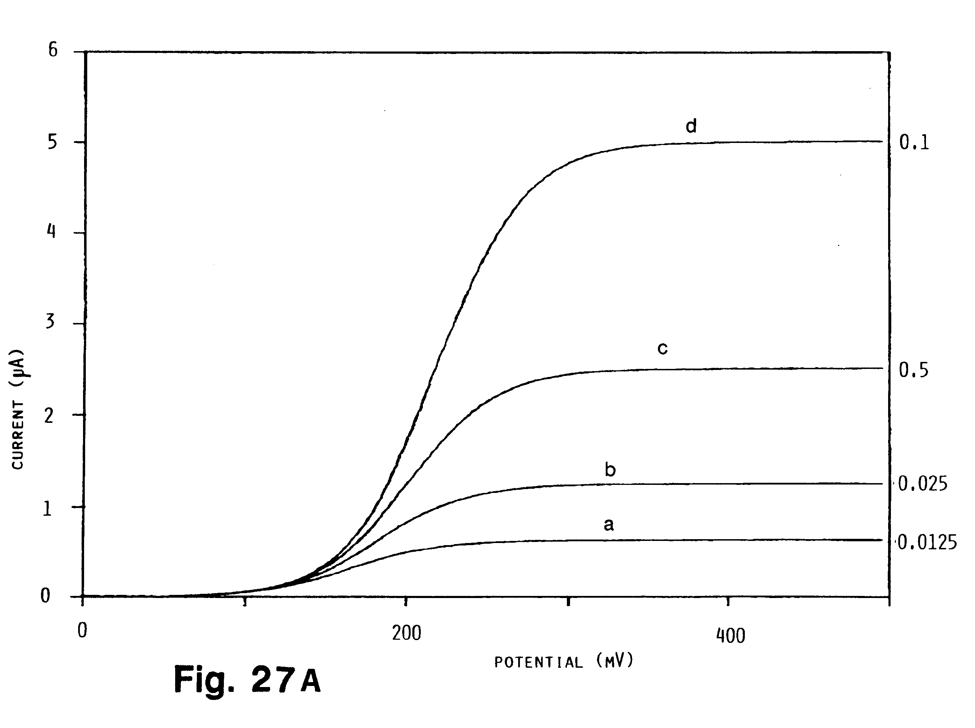

This behaviour can be calculated, and a closed-form solution can be obtained. Alternately, it can be simulated. A comparison between theory and experiment is shown in Fig. 27.

For an actual electrode, approximate values can be found for P and $M_p$ from the maximum value of $I_d$ and from its half-wave potential, $E_{d1/2}$ (E at $I_d(max)/2$). These two parameters characterize this part of the sensor electrode.

In the presence of glucose, the enzyme (GOD) begins to play a role. The exact mechanism of both oxidation of glucose and of electron transfer from the reduced form of the enzyme to the charge-transfer mediator is still unknown. However, the present data can qualitatively and quantitatively be, at least partly, explained by the following kinetic relation compatible with the known stoichiometry:

The glucose velocity (v):

$$v = \frac{V_{max}}{1 + K_m/[glucose] + K_f/[CTM^+]^2} \quad (3)$$

where $V_{max}$ is an amplitude parameter (i.e. a "maximum" velocity), and $K_m$ and $K_f$ are constants.

The catalytic current due to glucose oxidation is thus characterized by the above three parameters, $V_{max}$, $K_m$ and $K_f$. Only the first of these can be experimentally manipulated. The catalytic current is superimposed on the background current, $I_d$.

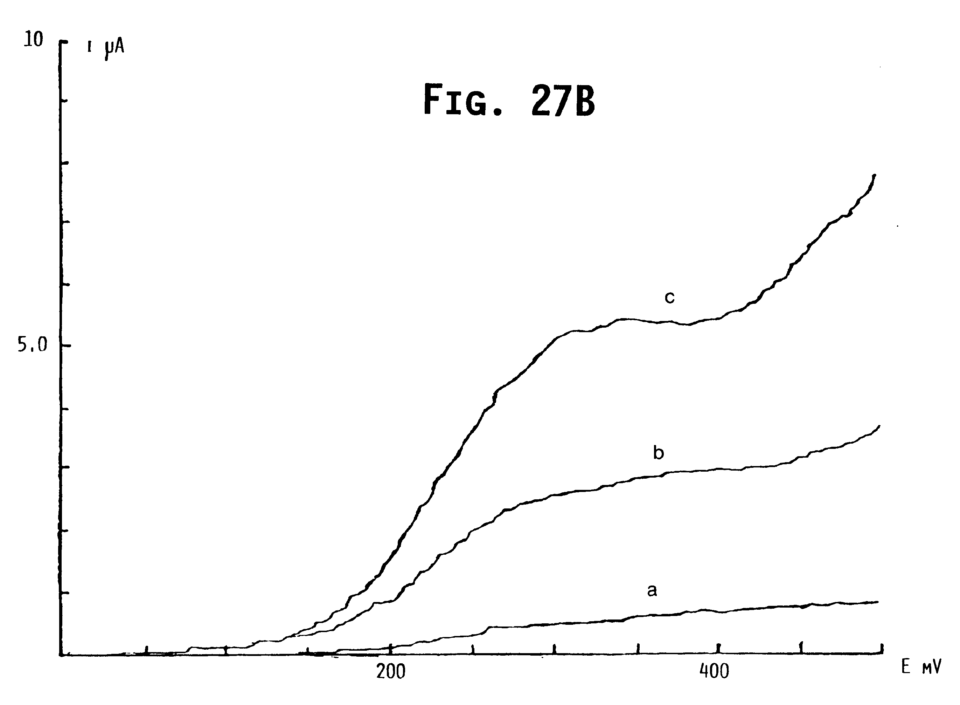

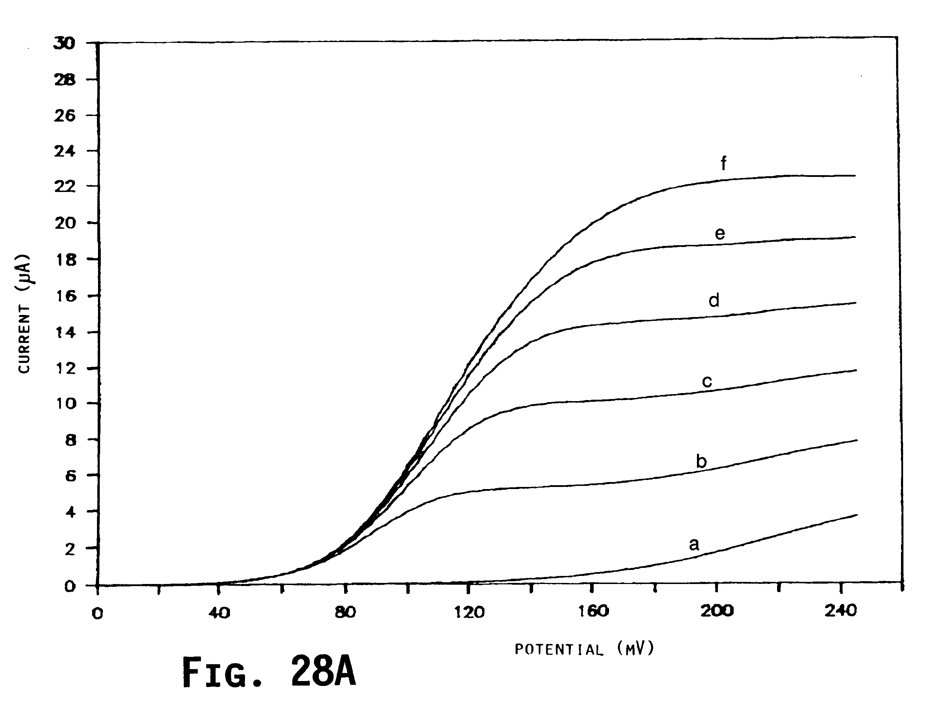

Fig. 28 shows the superposition of the catalytic current and the background current, both simulated and experimentally determined.

From Fig. 28 it is clear, that the catalytic current ($I_c$) builds up above the diffusional current, in other words when the latter has already reached a plateau value. From the above, it is apparent that under these conditions the concentration of CTM at the carbon surface ($M_c$) must be very low, and the resupplying of CTM from the PM phase is solicited at "full power", i.e. maximally, because the concentration gradient $M_p - M_c$ can no longer increase to cope with rising demand, for example in the case of convection in the aqueous medium of the sensor electrode surface. It can be shown, both theoretically and experimentally, that the reproducibility and/or the signal-to-noise ratio are poor in this operational range.

In order to obtain a much better reproducibility, it is necessary to separate, along the voltage abscissa, the catalytic wave from the diffusional wave. The former must develop at a lower voltage in a range in which the diffusional current is far from being at its maximum. Fortunately, this can easily be achieved by increasing the charge-transfer mediator concentration. The $E_{d1/2}$ for $I_d$ is concentration independent, whereas $E_{c1/2}$ (E at $I_c(max)/2$ for $I_c$ moves towards lower potentials when the charge-transfer mediator concentration increases. This point is exceedingly important. The separation of $I_c$ from $I_d$ is by far the most important aspect of this sensor electrode principle. Put in another way, reliability is achieved by replacing the usual regulation of $CTM^+$ concentration by means of potential variation by a judicious choice of the preparation of the pasting material.

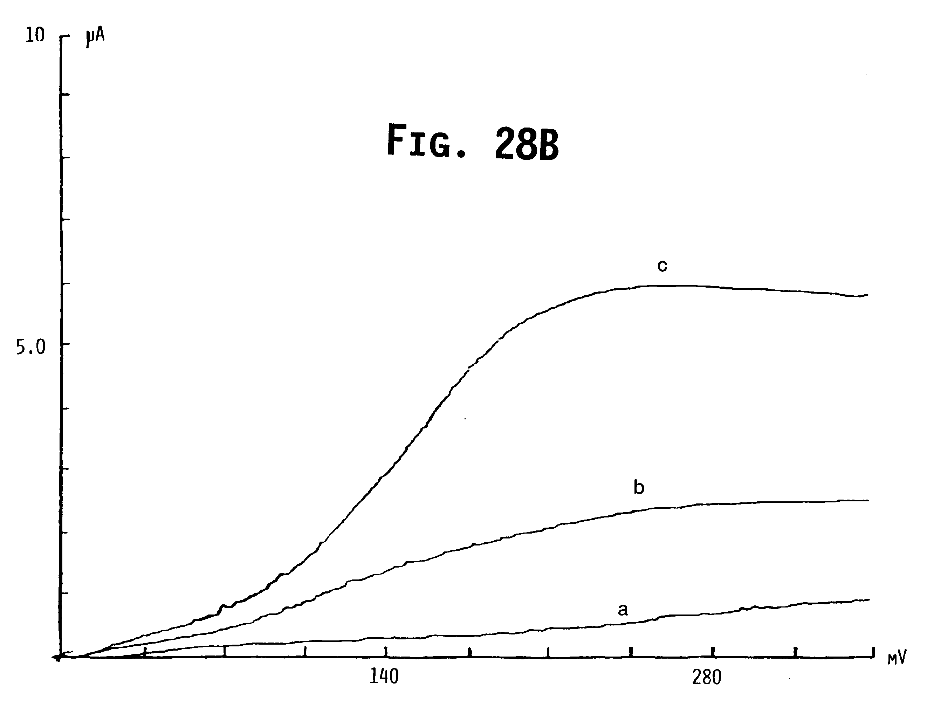

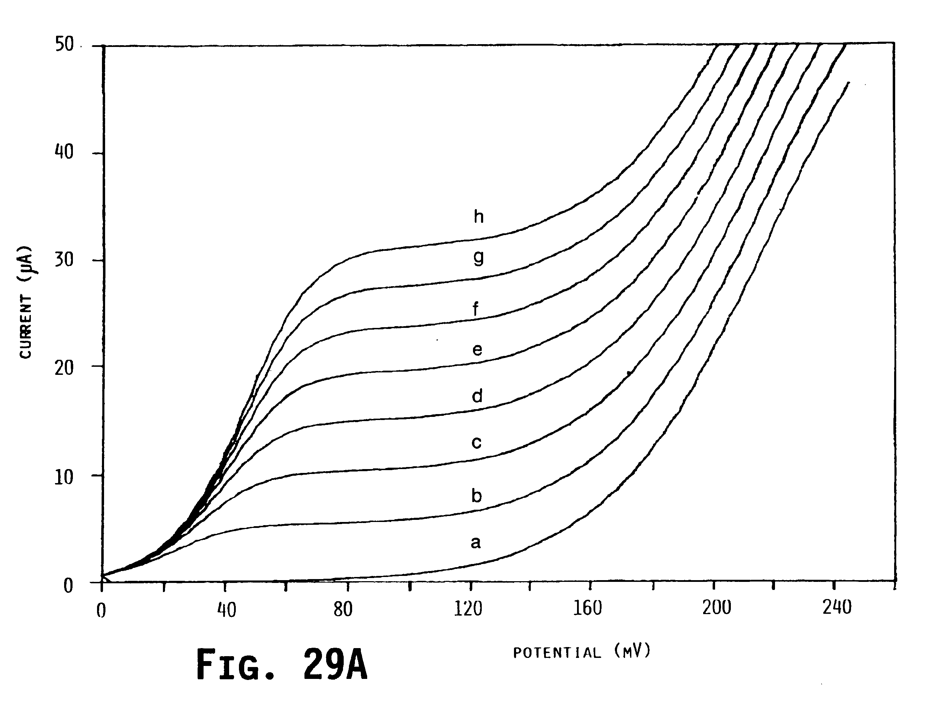

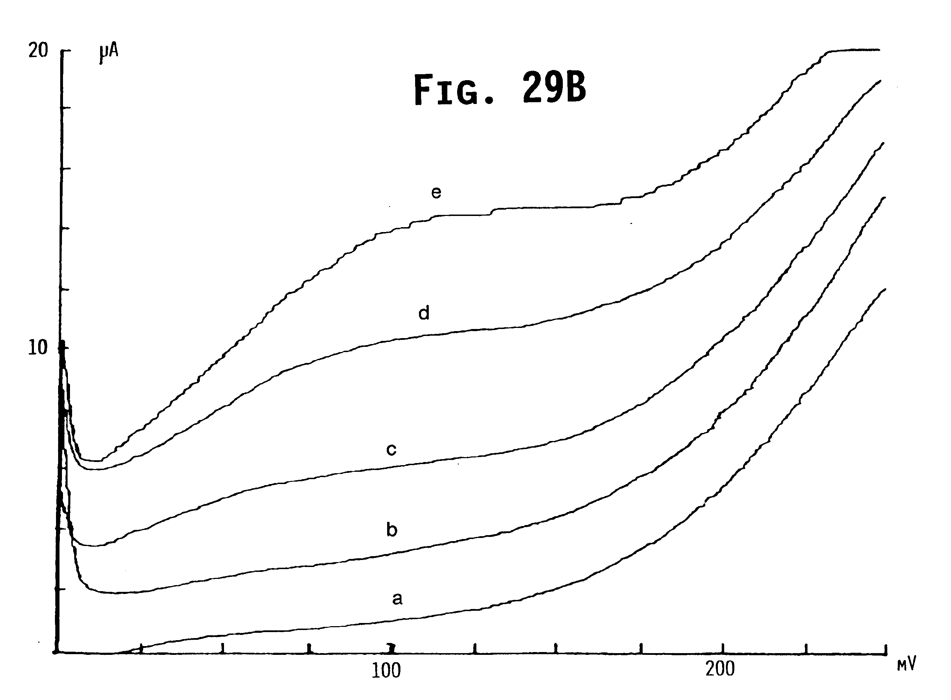

This separation of $I_c$ from $I_d$ is demonstrated both experimentally and in simulation in Fig. 29.

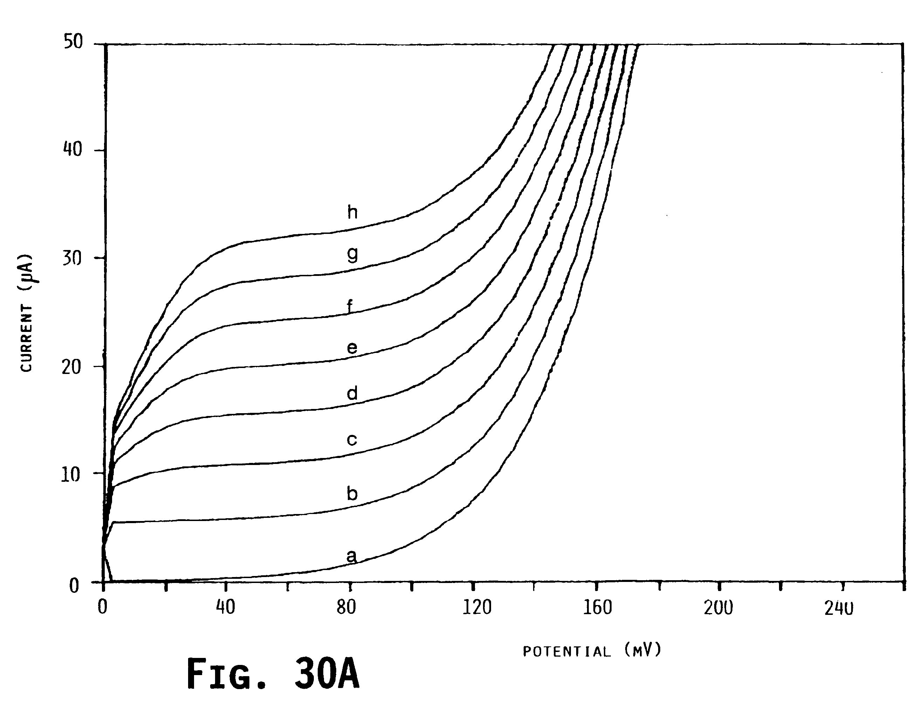

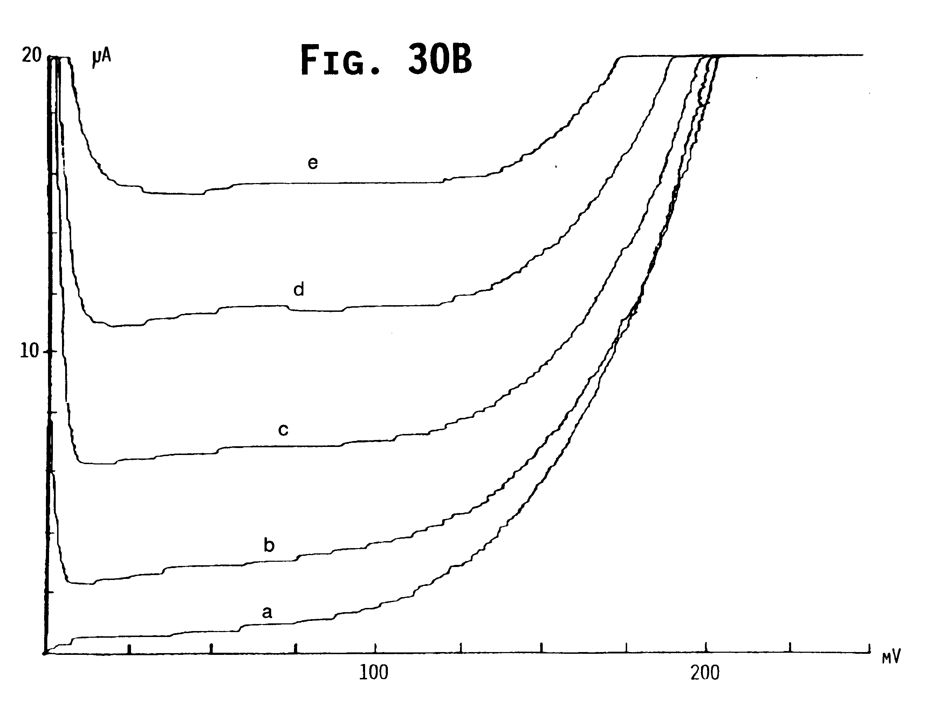

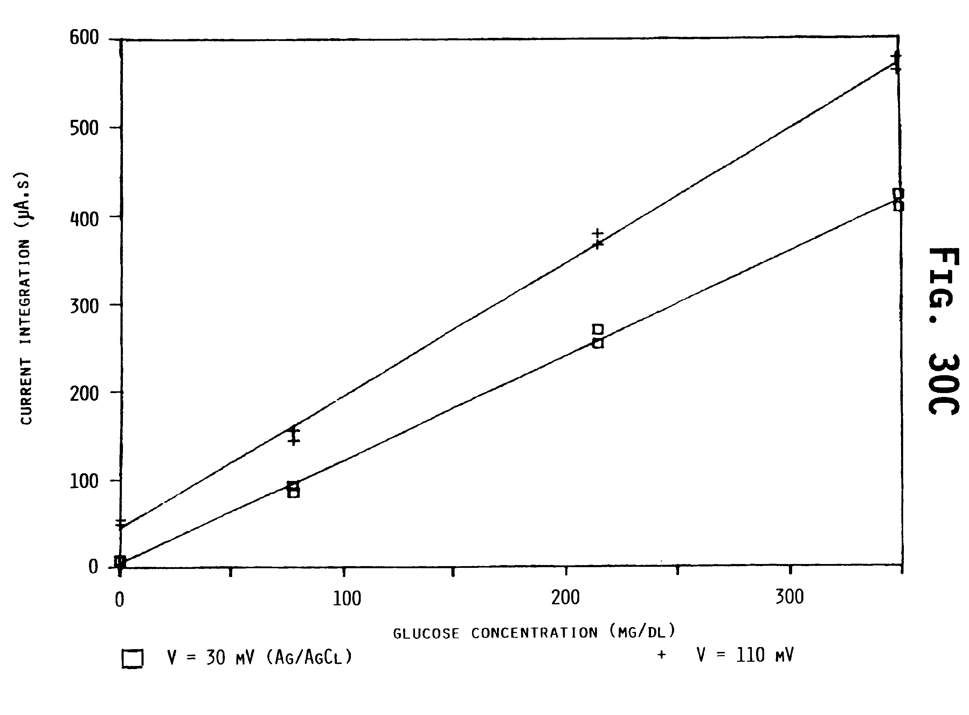

Two important advantages of this technique are the very low background current at zero glucose concentration allowing a "single-point" calibration and leading to improved linearity (at high $[CTM^+]$), as can be expected from relation (3) and is apparent from Fig. 30.

The specifications using the present techniques are:

(with 1,1′-dimethylferrocene as CTM, and with an Ag/AgCl reference electrode operating at 145 meq/l of chloride, i.e. at a chloride concentration not substantially different from that found in normal human whole blood plasma)

$E_{d1/2}$ for $I_d$      225 mV      upper limit      225 + 20 mV

                         lower limit      225 - 50 mV

$E_{c1/2}$ for $I_c$      lower than 0

Preferred working voltage 20-100 mV, in particular 20-30 mV; operating range 20 to 200 mV

The partition coefficient of 1,1′-dimethylferrocene between paraffin oil and pure water is found to be in the range of 1000-5000.

## Method to ensure that the charge-transfer mediator is present in the sensor electrode in a sufficient amount

The amount of the charge-transfer mediator present in the sensor electrode is a very important feature of the present invention. The amount should throughout the life time of the electrode device be sufficiently high to ensure that the concentration of the oxidized form of the charge-transfer mediator in the aqueous sample is substantially constant during the measurement.

Further to the specifications given above of the sensor electrode according to the invention, the following method may be used to ensure that a sufficient amount of 1,1′-dimethylferrocene is present in the sensor electrode.

The availability of a sufficient amount of the reduced form of the charge-transfer mediator, e.g. 1,1′-dimethylferrocene can be assessed by the electrode current generated when measuring on a standardized glucose-free sample, such as, e.g., polyvinylpyrrolidone solution. The generated flow current follows a Nernstian relationship and after correction for the current of the background, the relationship is approximately

$$\frac{I_{x+25\,mV}}{I_x} = e$$

(where e is the base of natural logarithm)

on sweeping the potential over the range of 0 to 250 mV ($0 < x \leqq 225$ mV). The potential given is the potential between the sensor electrode and a suitable reference electrode such as a Ag/AgCl electrode, and further characterized in that the measurement is performed at a potential where the absolute value of the slope of a plot of the current versus potential is substantially zero and less than 0.01 $\mu A/mv$.

The invention will now be further described with reference to the drawings, wherein

Fig. 1 is a perspective view of a first embodiment of the measuring apparatus according to the invention,

Figs. 2 and 3 are side views in an enlarged scale of an electrode assembly or electrode device having a cutter counter member mounted slidably thereon,

Fig. 4 is a top plan view of the electrode assembly or electrode device shown in Figs. 2 and 3,

Figs. 5 and 6 are side views and partially sectional views of the apparatus shown in Fig.1 with the lid member in an open and closed position, respectively,

Fig. 7 is a top plan view of the apparatus wherein the lid member and certain wall parts have been removed to show a ratchet mechanism,

Fig. 8 is a side view illustrating an electrode assembly as shown in Figs. 2-4 being inserted into the apparatus shown in Figs. 5-7,

Fig. 9 is a fragmentary sectional view of the apparatus shown in Figs. 5-7 illustrating a skin puncturing mechanism of the apparatus in an enlarged scale,

Fig. 10 is a side view of the apparatus illustrating how a driving spring of the skin puncturing mechanism may be tightened,

Fig. 11 is a perspective view of a second embodiment of the measuring apparatus according to the invention,

Figs. 12-18 illustrate a sequence of operational steps of the apparatus during use, and

Fig. 19 is the display of the measuring apparatus shown in an enlarged scale.

Fig. 20 is an overall schematic and block diagrammatical view of a prototype implementation of an electronic circuitry of an apparatus according to the present invention,

Figs. 21-24 are block diagrammatical view of electronic circuities of individual sections of the prototype implementation shown in Fig. 20.

Fig. 25 is a diagrammatical view of responses generated by means of a test bench apparatus illustrating the dependency of current responses generated by an apparatus according to the present invention as a function of on the one hand a polarizing voltage supplied to two electrodes of the apparatus and on the other hand the content of blood glucose in samples,

Fig. 26 is a diagrammatical view of two response curves generated by two sensor electrodes constituting two components of an electrode device of the apparatus according to the present invention.

Fig. 27A and 27B are theoretical and experimental curves, respectively, of potential versus different current for various concentrations of 1,1'-dimethylferrocene,

Fig.28A and 28B are theoretical and experimental curves, respectively, of potential versus diffusional plus catalytic current for various concentrations of glucose,

Fig. 29A and 29B are theoretical and experimental curves, respectively, of potential versus diffusional plus catalytic current for various concentrations of glucose,

Fig. 30A and 30B are theoretical and experimental curves, respectively; of potential versus diffusional plus catalytic current for various concentrations of glucose, and

Fig. 30C shows calibration curves for glucose concentration in human whole blood versus integrated current at two different applied potentials.

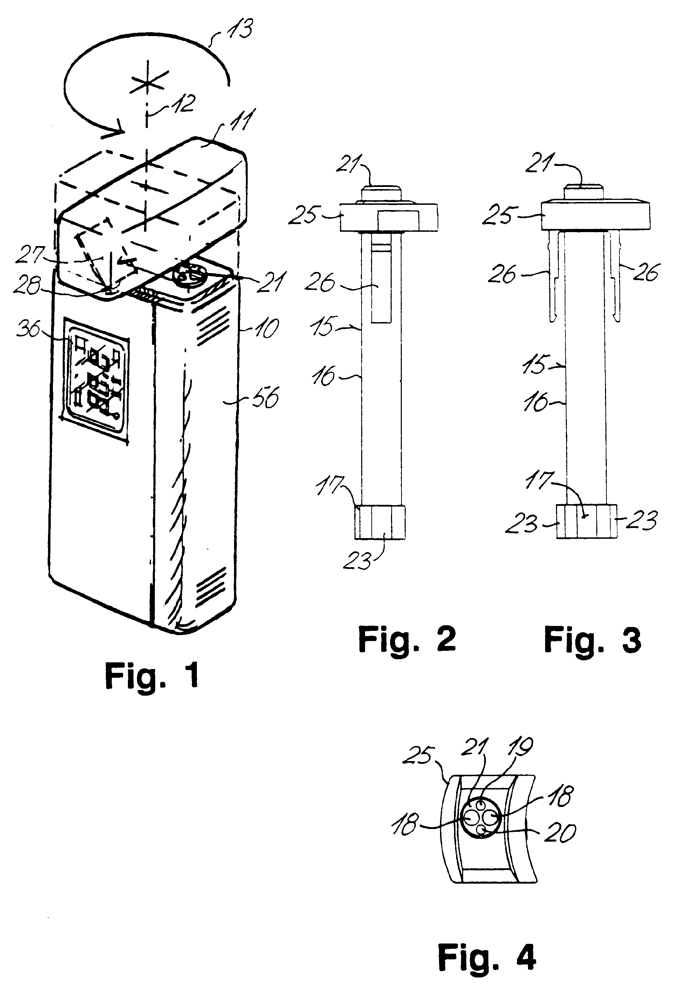

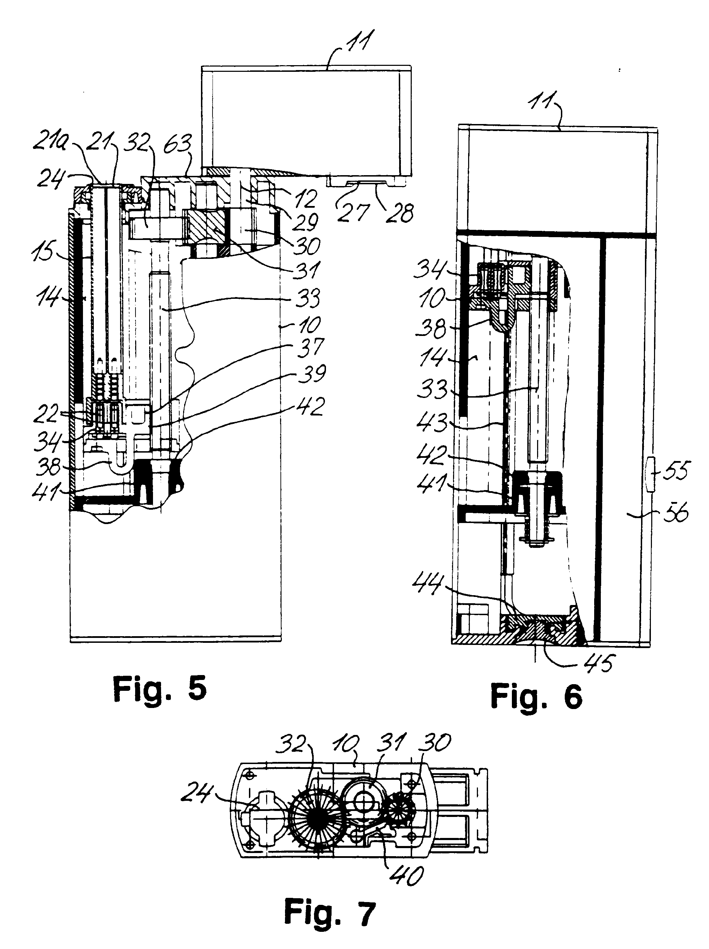

The measuring apparatus shown in Figs. 1 and 5-7 comprises a frame part or housing 10 and a lid-like member 11 mounted on the housing 10 so as to be rotatable in relation thereto about an axis 12 as indicated by an arrow 13 in Fig.1. The housing 10 as well as the lid-member 11 may be made from two or more separately manufactured parts which may, for example, be made from plastics material by die casting. The housing 10 defines a cavity 14 (Figs. 5 and 6) therein for receiving an electrode device or electrode assembly 15 as that shown in Figs. 2-4.

The electrode assembly shown in Figs. 2-4 comprises a cylindrical, rod-shaped electrode body member 16 having a collar-like enlargement 17 at one end. The electrode body member 16 may be made from plastics material or another electrically insulating material, and a number of mutually spaced, rod-shaped electrodes 18-20 are embedded in the electrode body member 16 and extend longitudinally through the electrode body member 16 from an end surface 21 opposite to the enlargement 17. Each of the electrodes 18-20 is connected to a terminal pin 22. The terminal pins 22, which are arranged within the collar-like enlargement 17, form an electrical connector plug. The electrodes each having a free end surface exposed at the end surface 21 of the electrode body member 16, may comprise a pair of sensor electrodes 18, a reference electrode 19, and a counter electrode 20. Each of theses rod-shaped electrodes 18-20 has a substantially uniform cross-section at least along a substantial part of the length of the electrode assembly 15 from the end surface 21.

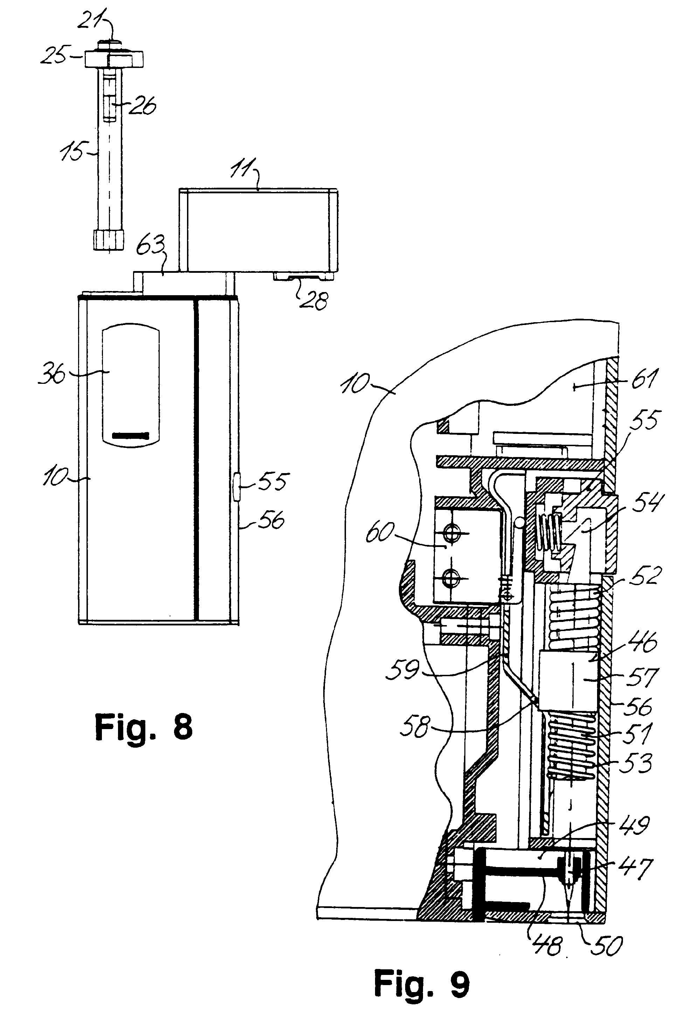

The collar-like enlargement 17 of the electrode assembly 15 has radial projections 23 formed thereon, and an inlet opening 24 defined in the top wall of the housing 10 (Fig. 7) has a contour corresponding to the outer contour of the enlargement 17 so that the electrode assembly 15 may be inserted into the cavity 14 through the inlet 24 in the correct rotational position only. Before the electrode assembly is mounted in the cavity 14 of the housing 10, the cylindrical end portion of the electrode assembly is inserted through a circular opening defined in a cutter counter member 25 as shown in Figs. 2-4. The counter member 25 has a pair of axially inwardly extending legs 26 having radial outer surfaces being substantially aligned with the radial outer surfaces of the projections 23 of the enlargement 17. Thus, when the electrode assembly 15 with the counter member 25 mounted thereon is inserted into the cavity 14 through the inlet opening 24 as illustrated in Fig. 8, the bottom surface of the counter member 25 is positioned in engagement with the upper wall surface of the housing 10, and the end surface 21 of the electrode assembly 15 is substantially aligned with or extends slightly from the upper surface of the counter member 25. A cutting member or cutting blade 27 having a cutting edge 28 is mounted in the rotatable lid member 11 in such a position that cutting edge 28 is arranged substantially in the plane defined by the upper surface of cutter counter member 25 and that the cutting edge 28 is moved along a circular path intersecting the electrode assembly 15, when the lid member 11 is rotated as indicated by the arrow 13 in Fig. 1. This means that rotation of the lid member 11 causes that a thin slice is cut from the upper end portion of the electrode assembly 15 if such end portion extends slightly beyond the outer surface of the cutter counter member 25. In fact, the apparatus is adapted to move the electrode assembly 15 longitudinally outwardly by a small increment each time the lid member 11 is rotated 360°, so that a thin slice will be cut from the outer end of the electrode assembly for each rotation of the lid member 11 as will be described in more detail with reference to Figs. 5-7.

As shown in Fig. 5 the lid member 11 is fastened to the outer end of a shaft 29 defining the axis of rotation 12 and having a driving gear 30 mounted on its inner end. The gear 30 is meshing with an idle gear 31 which in turn is meshing with a driven gear 32 mounted on one end of a screw spindle 33 rotatably mounted within the housing 10 and extending substantially parallel with the electrode assembly 15 inserted in the cavity 14. An electrical connecter socket 34 is arranged in the cavity 14 and is aligned with the inlet opening 24 so that the terminal pins 22 of the electrode assembly are received in the socket 34 when the electrode assembly 15 is inserted into the cavity 14 as described above. This means that the electrodes 18-20 are electrically connected to an electronic measuring circuitry 35 arranged within the housing 10 as diagrammatically indicated in Fig. 6. Such measuring circuitry may comprise a visual display 36.

The socket 34 is connected to a screw engaging member 37 through a flexible U-shaped connecting part 38. The screw engaging member has a concave threaded surface part 39 forming part of a cylinder surface and being biassed into engagement with the outer threads of the screw spindle 33 by the connecting member 38.

Rotation of the lid member 11 also causes rotation of the driving gear 30, whereby the screw spindle 33 is rotated via the gears 31 and 32. The thread engagement between the screw spindle 33 and the screw engaging member 37 causes displacement of the engaging member 37 and of the socket member 34 and consequently of the electrode assembly 15 in its longitudinal direction, when the screw spindle 33 is caused to rotate by rotating the lid member 11. A pawl 40 (Fig. 7) may engage with one of the gears 30-32 so as to form a ratchet mechanism permitting rotation of the lid member 11 in only one direction, namely in the direction causing an upward movement of the socket 34. The gear ratio may be chosen so that the slice cut from the free end of the electrode device 15 has a desired thickness. In the presently preferred embodiment the gear ratio is chosen so that one rotation of the lid member 11 causes an upward movement of the electrode assembly 15 by an increment corresponding to half the pitch of the screw thread of the screw spindle 33. Thus, the increment or the slice thickness may, for example, be 0.2-0.4 mm, preferably 0.3 mm.

The lower end of the screw spindle 33 may be journalled in a bearing block 41, and the bottom surface of a cam disc 42, which is fastened to the screw spindle 33, is in engagement with an adjacent upper cam surface of the bearing block. The cooperating cam surfaces are shaped so as to move the screw spindle 33 and consequently the socket 34 and the electrode assembly 15 slightly axially between an extended position in which the upper end surface 21 of the electrode assembly 15 extends slightly above the upper outer surface of the counter member 25, and a retracted position in which the end surface 21 of the electrode assembly 15 is slightly retracted, for example about 1 mm, in relation to the outer surface of the counter member 25 so that the end surface 21 forms the bottom of an upwardly open, cup-shaped cavity or measuring chamber 21a as shown in Fig.5. The interengaging cam surfaces are shaped so that the end portion of the electrode assembly 15 is retracted when the lid member 11 is in its open position as shown in Figs. 5, 8 and 14-17, and in its extended position when the cutting edge 28 of the cutting blade 27 passes the electrode assembly 15.

As will be described in more detail below the lid member 11 is rotated so as to cut a thin slice from the free upper end portion of the electrode assembly 15 before exposing the end surface 21 of the electrode assembly to a liquid sample to be measured. This means that the electrode assembly 15 is eventually consumed so that it must be replaced when a number of measurements have been made and the socket 34 has been moved to an upper position within the cavity 14 of the housing as shown in Fig. 6. In this position the free, hook-shaped ends of the legs 26 of the counter member 25 are engaging behind the enlargement 17 of the electrode assembly 15 so that the remaining part of the electrode assembly 15 may be removed from the cavity 14 in the housing 10 by removing the cutter counter member 25 from the upper wall of the housing. Before a new electrode assembly 15 may be inserted in the cavity 14 the socket 34 and the screw engaging member 37 connected thereto by the connecting part 38 must be returned to its lower position. One end of a string

EP 0 478 550 B1

member 43, such as a fishing line or cord, is connected to the engaging member 37 or the connecting part 38, and the other end portion of the string member 43 is wound on a winding member 44 which is rotatably mounted on the inner surface of the bottom wall of the housing 10. The winding member 44 is connected to a screw member 45, which extends through the bottom wall of the housing 10 and is provided with an outer slot shaped so that the winding member may be rotated in one direction only, for example by means of a coin. When the winding member is rotated in said direction, the string member 43 is wound unto the winding member, whereby the string member is tensioned so as to apply a downwardly directed force to the screw engaging member 37. Because the space defined between the socket 34 and the engaging member 37 is no longer occupied by the enlargement 17 of the electrode assembly 15, the screw engaging member 37 may flex out of engagement with the screw threads of the spindle 33 so that the socket 34 and the engaging member 37 may be moved downwardly to the lower position shown in Fig. 5. Now, a new electrode assembly 15 may be mounted in the cavity 14 of the housing 10 in the manner described above.

As described more detailed below the measuring apparatus may be used for measuring a constituent of a small liquid sample which may be arranged in the cup shaped measuring chamber defined by the end surface 21 of the electrode assembly 15 and by the surrounding wall parts of the cutter counter member 25. The liquid samples to be measured may be body liquids or liquids of any other type. However, the preferred embodiment of the apparatus is adapted to measure a constituent of a blood sample. Thus, the apparatus may be used by diabetics for determining the content of glucose in a blood sample, which may be a single drop of blood. Therefore, the apparatus comprises a skin puncturing mechanism 46 for puncturing the skin of the user so as to collect a drop of blood from the puncture. This skin puncturing mechanism is shown in Fig. 9. A skin puncturing needle 47 is mounted at the free end of a flexible arm 48 within a needle chamber 49. The pointed end of the needle is arranged within the chamber 49 opposite to an opening 50 formed in the bottom wall of the housing 10. An impact pin 51 aligned with the needle 47 is mounted so as to be axially displaceable under the influence of a strong compression spring 52 surrounding the upper end portion of the pin 51 and a weaker compression spring 53 surrounding the lower end of the pin 51. An upper hook-shaped end 54 of the pin 51 may engage with a depressible button 55 mounted in a housing wall part 56. In this engaging position, which is shown in Fig. 9, the strong compression spring 52 having its ends in engagement with the depressible button 55 and an enlargement 57 of the impact pin 51, respectively, is fully tightened, while the weaker compression spring 53 biassing the pin 51 in the opposite direction, is released. The enlargement 57 of the pin cooperates with a cut-out 58 in a bracket 59 of plate metal fastened to the housing 10, so as to limit the downward movement of the impact pin 51.

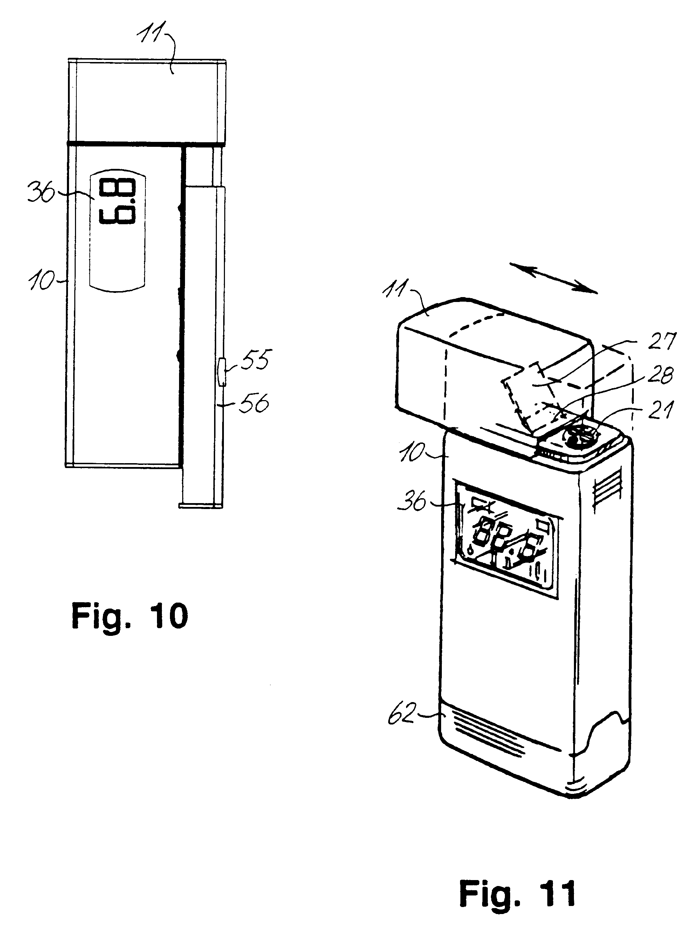

When the pin 51 is in the position shown in Fig. 9, the user may place the tip of his finger so as to cover the opening 50 in the bottom wall of the housing 10. Thereafter, the button 55 is depressed and brought out of engagement with the hook shaped end 54. Now, the impact pin 51 is suddenly moved downwardly under the influence of the spring bias of the compression spring 52. When the lower end surface of the pin 51 hits the upper end of the needle 47, the flexible arm 48 is bent so that the pointed end of the needle is moved outwardly through the opening 50, whereby the skin of the finger tip is punctured by the needle, and a drop of blood to be measured may be collected from the puncture. When the skin puncturing mechanism 46 has been fired, the wall part 56 and the depressible button 55 mounted therein in displaced downwardly as illustrated in Fig. 10. The downward movement of the button 55 in relation to the pin 51 causes tightening of the compression spring 52 and re-engagement of the hook shaped end with the button 55. When the displaceable wall part 56 is moved back from the position shown in Fig. 10 to its starting position, the mechanism 46 is ready for further use. The downward movement of the displaceable wall part 56 may cause activation of a micro switch 60 which may send a signal to the electronic measuring circuitry 35, so that the measuring result of the last measuring may be shown on the display 36. The electronic circuitry 35 may be powered by a battery (not shown) arranged in a battery chamber 61 formed in the housing 10, Fig. 10.

It should be understood that an outer end section or slice may be removed or cut from the electrode assembly 15 in any suitable manner before a measurement is made. Fig. 11 shows a modified embodiment of the measuring apparatus, wherein the lid-like member 11 is reciprocatingly movable in relation to the housing 10 rather than rotatable. Also in this case a mechanism for incremental moving the electrode assembly 15 axially outwardly for each reciprocating movement of the lid member 11 should be provided. In the embodiment shown in Fig. 11 the battery chamber may be defined in the bottom part of the housing 10, and access to the battery chamber may be obtained by removing a hood-shaped bottom part 62 of the housing. Otherwise, the apparatus shown in Fig. 11 may function similar to the apparatus described with reference to Figs. 1-10.

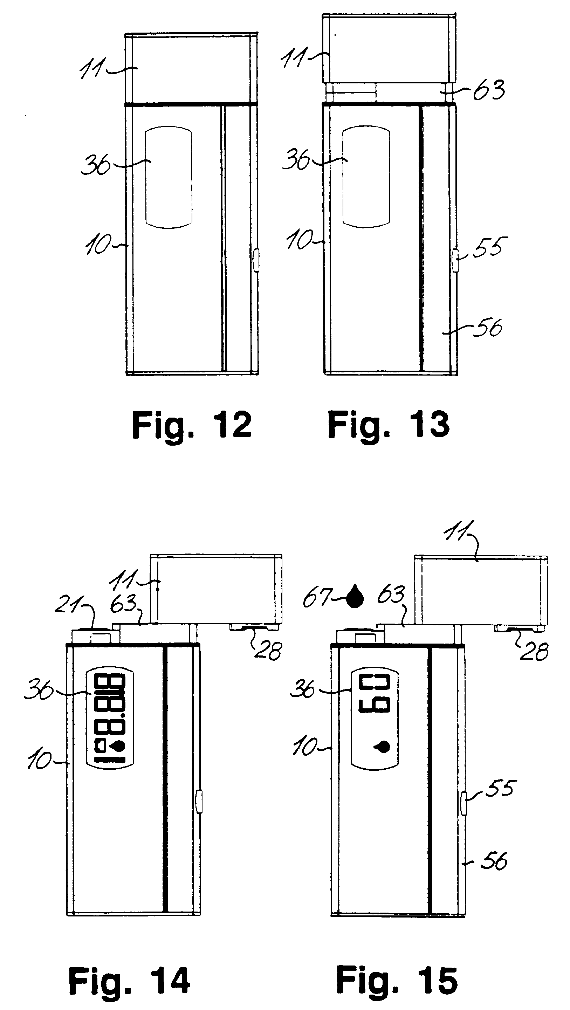

The use of the apparatus illustrated in Figs. 1 and 5-10 will now be described with reference to Figs. 12-18. As mentioned above, the electrode assembly 15 is being consumed during use of the apparatus because a thin slice is cut from the free end of the electrode assembly for each measurement. When the electrode assembly has been consumed, for example after about 100 measurements, or if the display 36 of the apparatus

16

indicates that the electrode assembly should be replaced, for example because it has been destroyed by a too high or too low storing temperature, the electrode assembly 15 is replaced by a new one as described above. Preferably, the cutting blade 27 is also replaceably mounted, and the cutting blade 27 may then also be replaced. The electrode assembly 15 or the cutter counter member 25 mounted thereon may contain coded information for calibrating the electronic measuring circuitry 35 of the apparatus to the type of the new electrode assembly 15. The apparatus may now be stored in the condition illustrated in Fig. 12, where the lid member 11 is in its fully closed position in which the exposed end surface 21 of the electrode device or electrode assembly 15 is protected. The apparatus may now be carried by the user in a pocket or a hand bag.

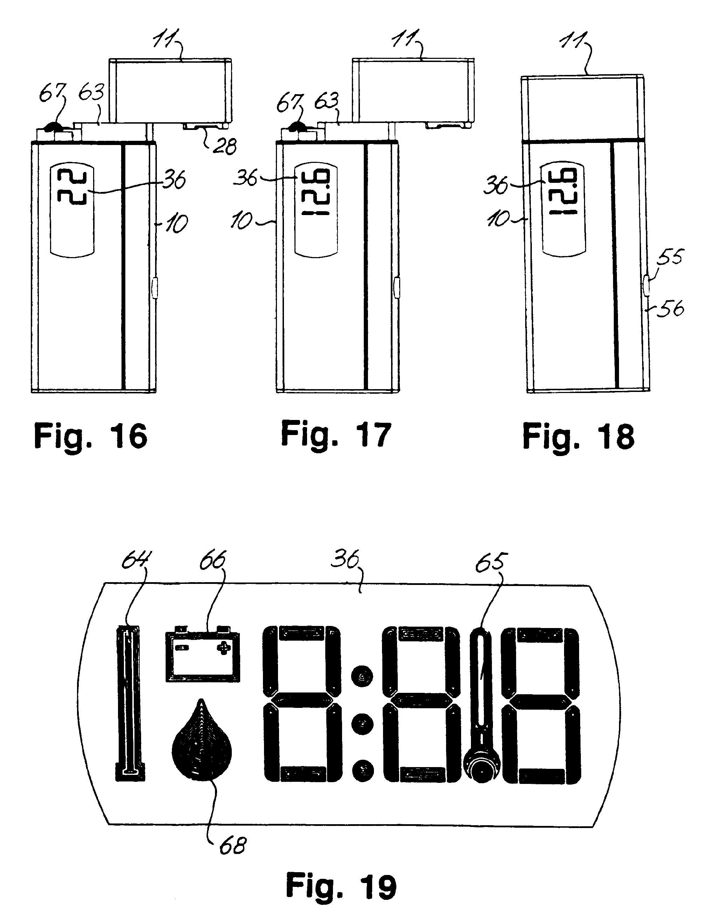

When a measurement is to be made, the lid-member 11 is moved axially outwardly to a position shown in Fig. 13 so as to move a lower skirt part of the lid member 11 out of engagement with a block shaped portion 63 extending upwardly from the top wall of the housing 10. The lid is now rotated 180° from the position shown in Fig. 13 to the position shown in Fig. 14, whereby the cutting edge 28 of the cutting blade 27 mounted in the lid member 11 intersects the upper free end portion of the electrode assembly 15 so that a thin slice having a thickness of for example 0.2-0.4 mm or about 0.3 mm is cut from the electrode assembly so that a fresh, non-contaminated exposed end surface 21 is provided. The rotating movement of the lid member 11 also causes that the electronic measuring circuitry 35 is energized and a visual indication appears on the display 36, Fig. 19. The display may indicate the waiting time in seconds as shown in Fig. 15. If the apparatus has registered an excessively high or excessively low storing temperature, for example a temperature above 40°C or below 0°C, since mounting of the electrode assembly 15 in the apparatus, a warning sign may appear on the display, for example by lightening a symbol 64, vide Fig. 19, and/or by a flashing symbol 65, and a new electrode assembly 15 must be mounted in the apparatus before a measurement can be made. In case the apparatus determines that the actual temperature is within an unacceptable range, for example between 0 and 15°C or between 35 and 40°C, this may be indicated by lightening of the symbol 65 on the display, and the measurement must be postponed till the actual temperature is within an acceptable range, for example between 15 and 35°C. If the voltage of the power source or battery of the apparatus is below an acceptable minimum limit, this may be indicated by a flashing symbol 66 on the display 36. In that case the battery must be replaced before a measurement can be made. If only the symbol 64 is lit, this may indicate that the remaining length of the rod-shaped electrode assembly 15 is below a certain limit, for example that the electrode length left is sufficient for less that ten measurements. If the electrode assembly 15 is completely consumed, the symbol 64 may be flashing, and a new electrode assembly must be mounted.

Provided that the display 36 indicates that the measuring conditions are satisfactory by lighting of the symbol 68 the user may puncture the skin on a finger tip by means of the skin puncturing mechanism 46 in a manner described above so as to provide a drop of blood. As described previously, after cutting the electrode assembly the fresh end surface 21 of the assembly may be slightly retracted, for example about 1 mm, in relation to the outer surface of the cutter counter member 25 so as to provide a small cup-shaped measuring chamber having a bottom formed by the end surface 21. The drop of blood 67 (25-50μl) may now be introduced into the measuring chamber as shown in Figs. 15 and 16 so that the end surface 21 of the electrode assembly 15 is covered thereby. In case the blood does not sufficiently cover the exposed end surfaces of all of the electrodes 18-20 this may be indicated by flashing of a symbol 68 of the display 36, and more blood must be supplied to the measuring chamber or the measurement procedure must be restarted. If the end surface 21 is sufficiently covered by blood, the symbol 68 is extinguished, and a count-down of the waiting time indication of the display 36 is initiated. When the waiting time indication has been counted down to zero, the waiting time indication is extinguished on the display and is replaced by an indication of the measuring result, for example the concentration of the constituent, such as glucose, being measured. In Fig. 17 the display 36 indicates that the glucose concentration in the drop of blood 67 is 12.6 mmol/l. When the measurement has been completed, the blood is removed from the measuring chamber, and the lid member 11 may be moved back to its closed starting position as shown in Fig. 18. The indication of the measuring result may remain at the display 36 for a certain predetermined period of time, for example five minutes. When a new measurement is to be made, the procedure described above is repeated, and before a new drop of blood is introduced into the measuring chamber a thin slice is cut from the free end portion of the electrode assembly 15 so as to provide fresh, uncontaminated electrode surfaces before the new liquid sample is introduced into the measuring chamber.

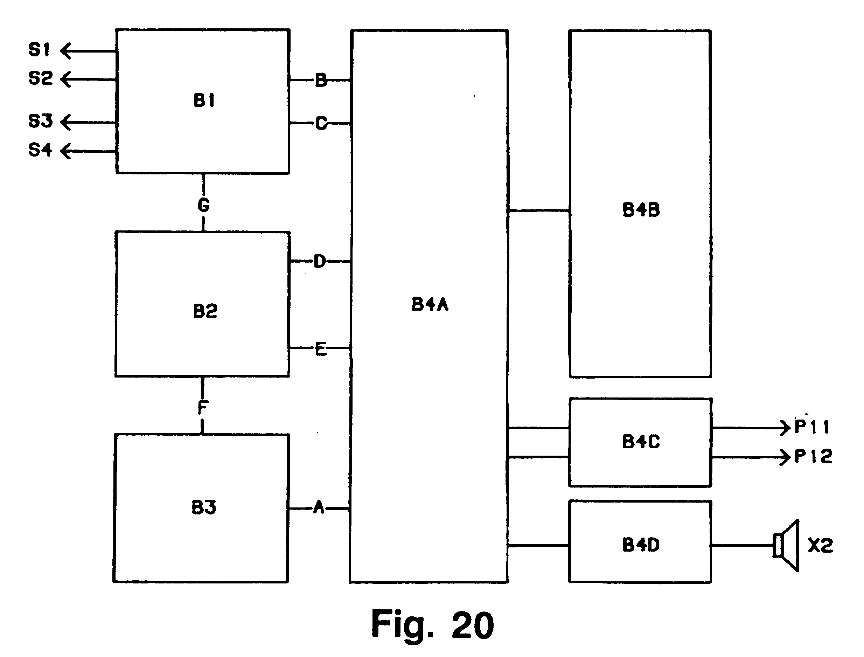

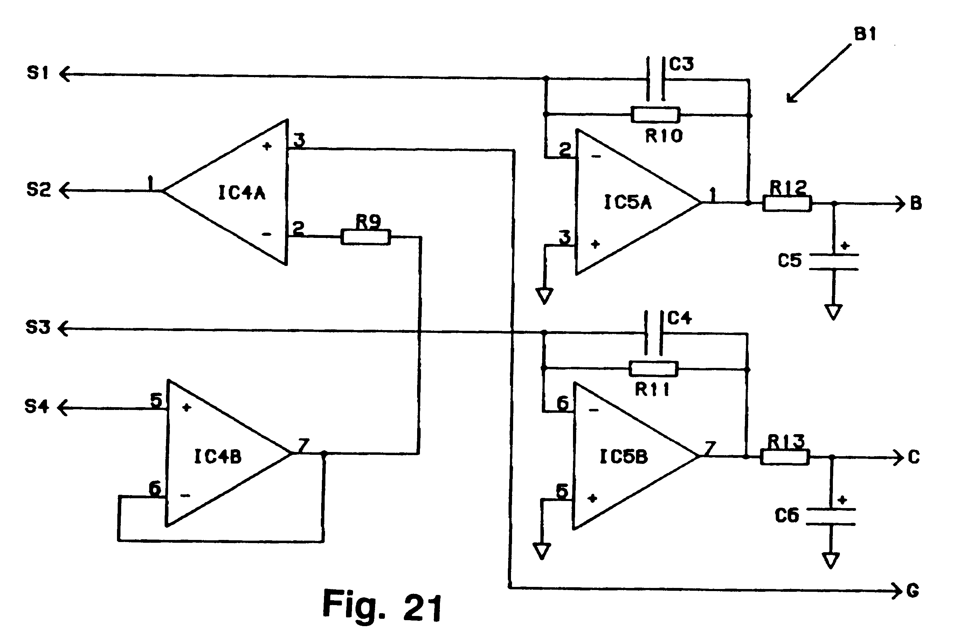

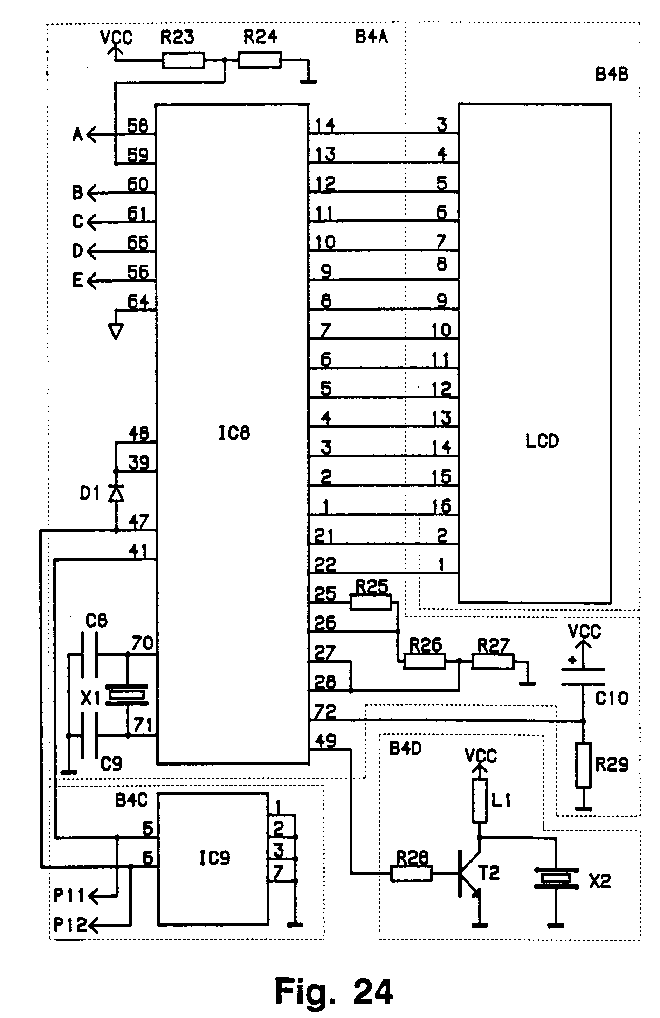

In Fig. 20 a block diagram of a prototype implementation of an electronic circuitry of the apparatus according to the present invention is shown. The electronic circuitry comprises a total of seven blocks designated B1, B2, B3, B4A, B4B, B4C, and B4D. The electronic circuitry is, as is evident from the above description of the method according to the present invention, connected to a total of four electrodes designated S1, S2, S3 and S4 of an electrode device. The electrodes S1, S2, S3 and S4 constitute a first measuring or sensor electrode, a current or counter electrode constituted by a silver rod, a second measuring or sensor electrode, and a reference electrode constituted by a chlorinated silver rod, respectively. The electrodes S1-S4 are, as is evident from Fig. 20, connected to the block B1, which constitutes an analog amplifying and impedance transforming section, which is shown in greater detail in Fig. 21, and which is connected through lines B, C, and G to the block B4A and the block B2, respectively. The block B2 constitutes a reference voltage generator block, which is shown in greater detail in Fig. 21, and which is further connected through lines D, E and F to the blocks B4A and B3, respectively. The block B3 is shown in greater detail in Fig. 24 and constitutes a temperature sensor section, which is further connected to the block B4A through a line A for supplying a temperature measuring signal to the block B4A. The block B4A is shown in greater detail in Fig. 24 together with the blocks B4B, B4C, and B4D and constitutes a central microprocessor controlling the overall operation of the apparatus. The block B4B constitutes a display section comprising a liquid crystal display, on which information is displayed to a person operating the apparatus. The block B4C constitutes a block into which data may be input through lines P11 and P12, which data may constitute additional data relevant to the operation of the apparatus, e.g. data representing parameters or characteristics of the components or materials of the electrodes S1-S4. The block B4D constitutes an audio or signalling driver block, which is connected to an audio signalling device, such as a loudspeaker or a buzzer $X_2$. The blocks B4B, B4C and B4D are connected to the central microprocessor block B4A exclusively, as is evident from Figs. 20 and 24.

In Fig. 21 a detailed block diagram of the block B1 is shown. The block B1 comprises a total of four operational amplifiers IC4A, IC4B, IC5A and IC5B. The operational amplifiers IC5A and IC5B constitute current voltage converters, which convert current signals generated by the sensor electrodes S1 and S3, respectively, into voltage signals supplied to the microprocessor block B4A through the lines B and C, respectively. The current voltage converters are constituted by inverters. Thus, the sensor electrodes S1 and S3 are connected to the inverting inputs of the operational amplifiers IC5A and IC5B, respectively. The non-inverting inputs of the operational amplifiers IC5A and IC5B are grounded. The output of the operational amplifier IC5A is connected to the inverting input of the operational amplifier IC5A through a feed-back resistor R10 and a capacitor C3. Similarly, the output of the operational amplifier IC5B is connected to the inverting input of the operational amplifier IC5B through a feed-back resistor R11 and a capacitor C4. The capacitors C3 and C4 serve the purpose of limiting the high-frequency gain of the operational amplifiers IC5A and IC5B. The output of the operational amplifier IC5A is connected to the line B through a low pass filter constituted by a resistor R12 and a capacitor C5. Similarly, the output of the operational amplifier IC5B is connected to the line C through a low pass filter constituted by a resistor R13 and a capacitor C6.

The block B1 comprising the operational amplifiers IC4A and IC4B further receives a reference voltage signal through the line G from the block B2, which voltage reference signal is input to the non-inverting input of the operational amplifier IC4A the output of which is connected to the current or counter electrode S2. The reference electrode S4 is connected to the non-inverting input of the operational amplifier IC4B which constitutes a unity gain voltage follower, as the output of the operational amplifier IC4B is connected to the inverting input of the operational amplifier IC4B through a short circuiting connection. The output of the unity gain voltage follower constituted by the operational amplifier IC4B is connected to the inverting input of the operational amplifier IC4A, which constitutes a high-gain differential amplifier. The operational amplifier IC4A serves the purpose of clamping the potential of the current or counter electrode S2 to a potential defined by the reference voltage signal supplied to the operational amplifier IC4A through the line G and further generating an output voltage signal in response to any voltage difference between the non-inverting input and the inverting input of the operational amplifier IC4A, which output voltage signal results in that current is supplied to the current or counter electrode S2. The reference voltage signal supplied to the non-inverting input of the operational amplifier IC4A through the line G is of the order of -110 mV. The current signals generated by the measuring or sensor electrodes S1 and S3 are within the range of 0-15 µA, and the voltage signals generated by the current voltage converters IC5A and IC5B are within the range of 0-2.5 V in response to a current signal of the above range of 0-15 µA.

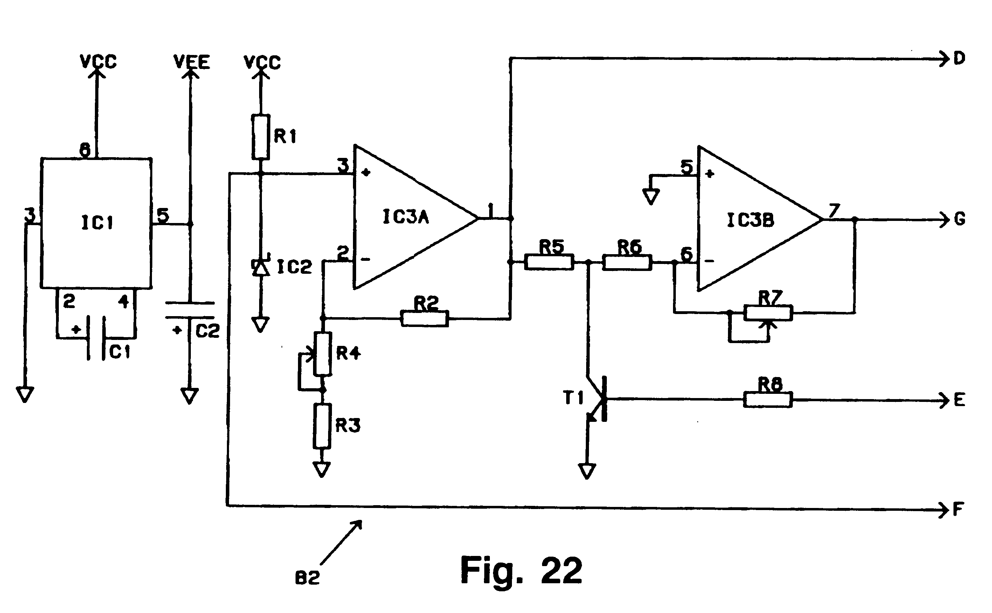

The reference voltage block B2 shown in greater detail in Fig. 22 comprises a voltage inverter circuit including an integrated electronic circuit IC1, which receives a positive supply voltage VCC at its terminal No. 8 and generates a negative supply voltage VEE at its terminal No. 5. The negative supply voltage VEE arises by charging of a capacitor C2 by discharging a capacitor C1 which originally is charged directly by the supply voltage. The positive and negative supply voltages VCC and VEE, respectively, are supplied to the active components of the electronic circuit, such as the above described operational amplifiers and the operational amplifiers to the described below.

The block B2 further comprises two operational amplifiers IC3A and IC3B which are connected in a cascade. The operational amplifier IC3A receives a positive reference voltage at its non-inverting input, which positive reference voltage is generated from the positive supply voltage VCC by means of a voltage reference circuit IC2, to which current is supplied through a resistor R1 from the positive supply voltage VCC. The reference voltage generated by the integrated circuit IC2 is output to the line F. The operational amplifier IC3A constitutes

EP 0 478 550 B1

a high gain non-inverting circuit, the output of which is connected to the inverting input of the operational amplifier IC3A through a feed-back resistor R2. The inverting input of the operational amplifier IC3A is grounded through a series configuration of a resistor R3 and a variable resistor R4 by means of which the gain of the operational amplifier IC3A may be set. The output of the operational amplifier IC3A is connected to the line D and further to the inverting input of the operational amplifier IC3B through a series configuration of two resistors R5 and R6. The node of the resistors R5 and R6 is connected to the collector of an NPN transistor T1, the emitter of which is grounded, and the base of which is connected to the line E through a resistor R8. By turning on the transistor T1, the node of the resistors R5 and R6 is grounded consequently grounding the inverting input of the operational amplifier IC3B. The non-inverting input of the operational amplifier IC3B is grounded, and the output of the operational amplifier IC3B is connected to the line G and further to the inverting input of the operational amplifier IC3B through a variable resistor R7, by means of which the gain of the operational amplifier IC3B may be set. The inverting operational amplifier IC3B generates a negative reference voltage at the line G from the positive reference voltage initially generated by the integrated circuit IC2 and amplified by the non-inverting operational amplifier IC3A. By grounding the node of the resistors R5 and R6 bridging the output of the operational amplifier IC3A and the inverting input of the operational amplifier IC3B, the negative reference voltage generated by the operational amplifier IC3B at the line G may be eliminated as the line G is shifted to ground potential when the node of the resistors R5 and R6 is grounded as the transistor T1 is turned on.

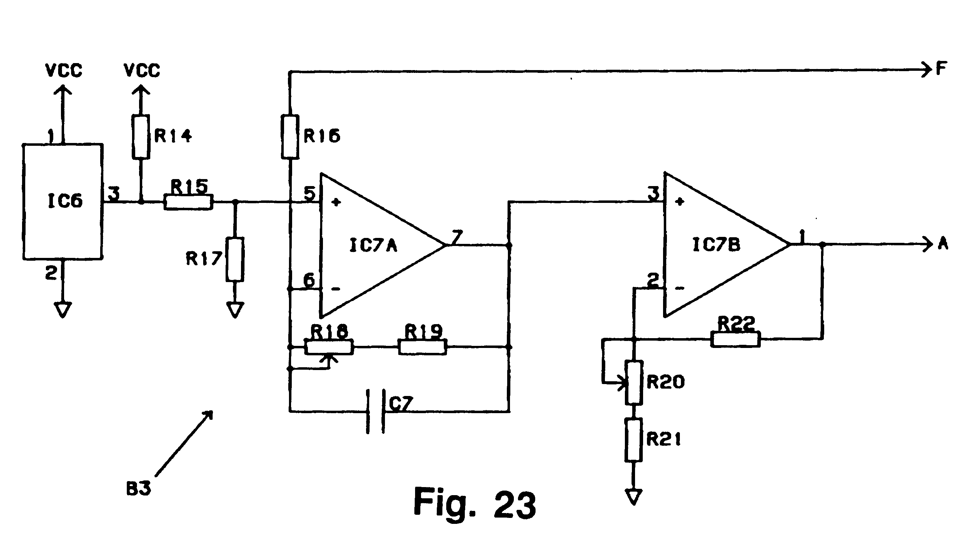

The block B3 shown in greater detail in Fig. 23 constitutes, as indicated above, a temperature sensor section including a temperature sensor constituted by an integrated electronic circuit IC6, which is arranged juxtaposed the electrode device body of the electrode device described above and which is connected to the positive supply voltage VCC and to ground. The integrated circuit IC6 generates at its output a DC-signal, which corresponds to the temperature detected by the integrated circuit. The output of the integrated circuit IC6 is connected to a non-inverting input of an operational amplifier IC7A through a resistor R15, which resistor R15 further constitutes a component of a voltage divider branch further comprising a resistor R14 and a resistor R17, which voltage divider branch interconnects the positive supply voltage VCC and ground for offsetting the output of the integrated circuit IC6 and the input of the operational amplifier IC7A at a positive offset voltage determined by the resistors of the voltage divider branch.

The output of the operational amplifier IC7A is connected to the inverting input thereof through a series configuration of a variable resistor R18 and a fixed resistor R19 and further a capacitor C7, which reduces the high-frequency gain of the operational amplifier IC7A. The inverting input of the operational amplifier IC7A is connected to the line F through a resistor R16 for receiving the reference voltage generated by the integrated circuit IC2 shown in Fig. 22. The operational amplifier IC7A generates at its output a voltage signal originating from the temperature representing signal generated by the integrated circuit IC6. The output of operational amplifier IC7A is connected to a non-inverting input of a second operational amplifier IC7B, which constitutes a high gain non-inverting amplifier circuit and has its output connected to its inverting input through a resistor R22, which inverting input is grounded through a series configuration of a variable resistor R20 and a fixed resistor R21. The output of the operational amplifier IC7B is further connected to the line A for presenting a voltage signal representing the temperature detected by the integrated electronic circuit IC6 to the micro-processor of the block B4A.

In Fig. 24, the blocks B4A, B4B, B4C and B4D are shown in greater detail. Within the block B4A an integrated circuit IC8 constituting a central control means implemented by a microprocessor is included. The microprocessor IC8 receives at respective inputs the temperature signal from the block B3 through the line A, the measuring signals from the block B1 through the lines B and C, the reference voltage from the output of the operational amplifier IC3A of the block B2 through the line D, and outputs a control signal through the line E from a respective output. The microprocessor IC8 includes an internal 8-bit analog/digital converter, in which the measuring signals received through the lines B and C are converted from analog form into digital form and compared to one another so as to determine whether the measuring signals differ from one another, and if so, whether the difference is beyond a preset acceptable threshold. The microprocessor also converts the temperature signal into digital form for calculating - in accordance with an integration routine to be described in greater detail below - a measuring signal on the basis of the measuring signals input to the microprocessor IC8 through the lines B and C and the temperature signal input through the line A. The microprocessor IC8 is further connected to an oscillator crystal X1 through respective terminals, which are further grounded through capacitors C8 and C9. Two resistors R23 and R24 serve as a voltage divider, so that the supply voltage can be monitored via the 8-bit analog digital converter. The microprocessor IC8 is further grounded through a respective terminal and generates at respective outputs display driver signals, which are supplied to the block B4B, which includes a liquid crystal display designated LCD.

The block B4C shown in the lower left-hand part of Fig. 24 includes a storage IC9 constituted by an $E^2PROM$

19

(Electrically Erasable Programmable Read Only Memory), which is connected to respective inputs of the microprocessor IC8 of the block B4A and further connected to the lines P11 and P12 for receiving data from an external data input source. In the prototype implementation, the data input lines P11 and P12 were used for inputting data to the $E^2$PROM IC9 representing characteristics of the electrodes of the electrode device for calibration of the measuring routine carried out within the microprocessor IC8 in calculating the measuring result on the basis of the measuring signals supplied to the microprocessor IC8 from the measuring or sensor electrodes of the electrode device through the block B1 and further the temperature signal supplied to the microprocessor IC8 from the block B3.

In the lower right-hand part of Fig. 24, the block B4D is shown including a buzzer constituting the loudspeaker $X_2$ shown in Fig. 20, which buzzer is connected to a switching transistor T2, the base of which is connected to a control terminal of the microprocessor IC8, the emitter of which is grounded, and the collector of which is connected to a first terminal of the buzzer and through and inductor L1 to the positive supply voltage VCC. The second terminal of the buzzer is grounded.

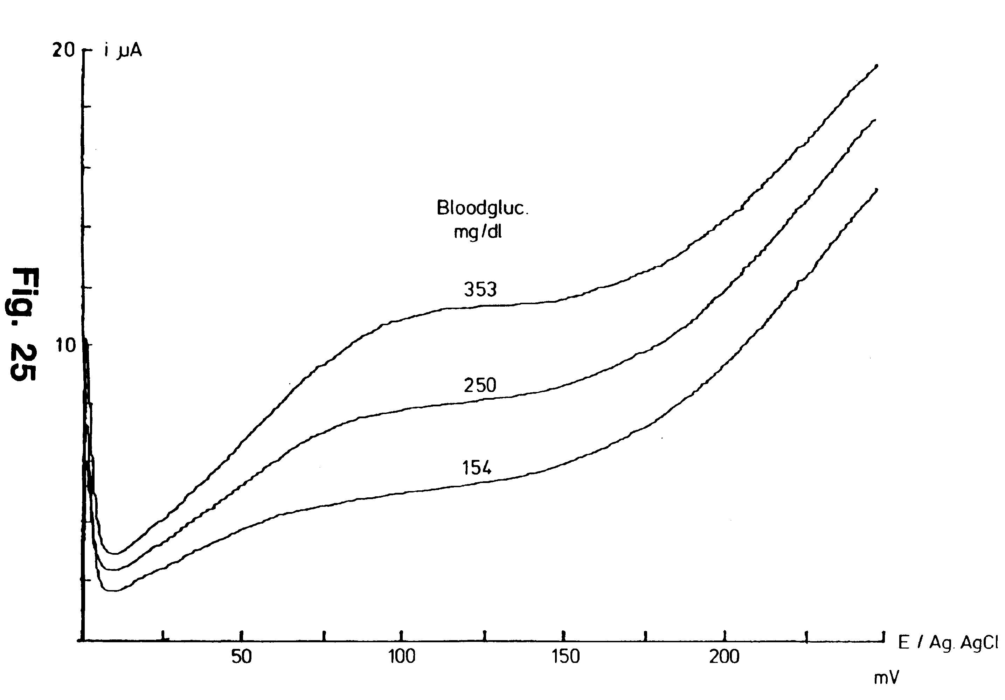

In Fig. 25 a diagram is shown comprising three response curves, which represent the current responses generated by a sensor or measuring electrode of an electrode device according to the present invention as a function of the polarizing voltage of the reference electrode relative to the current or counter electrode, i.e. the reference voltage supplied to the current or counter electrode and further as a function of the content of glucose in a blood sample as expressed in mg/dl. From Fig. 25 it is evident that a polarizing voltage of approximately 100-150 mV is an optimum polarizing voltage, as the response curves are substantially horizontal in this range due to the 0.1 M 1,1'-dimethylferrocene concentration in the paraffin oil of the sensor electrodes employed in Fig. 25. Thus, any current measuring result unambiguously represents a specific blood glucose content irrespective of any variation of the polarizing voltage.

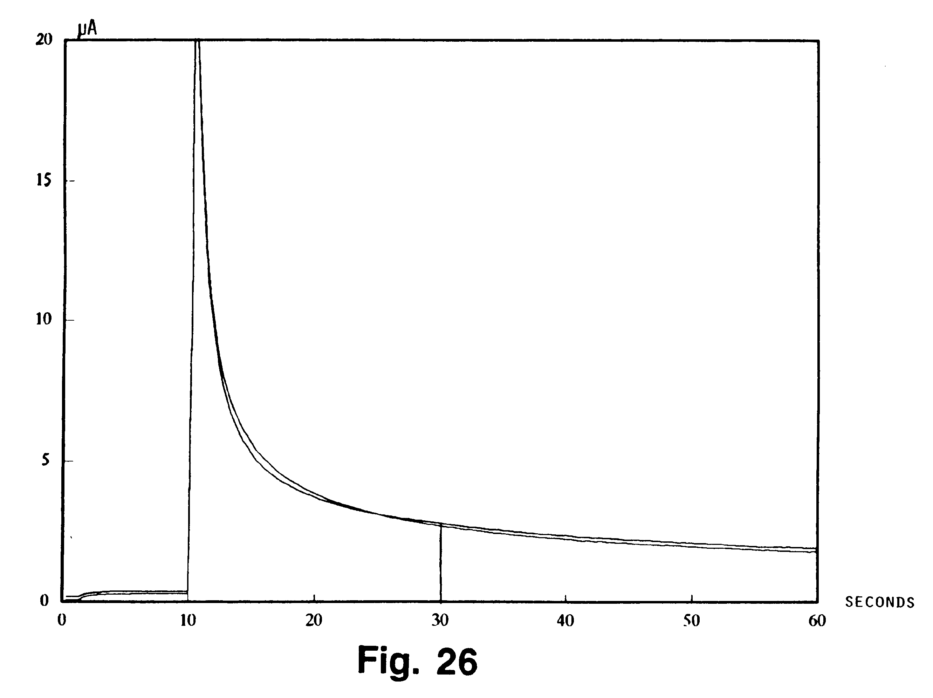

In Fig. 26, two response curves of two measuring or sensor electrodes of an electrode device according to the present invention are shown. Fig. 26 further illustrates the measuring routine carried out by the microprocessor of the electronic circuitry. For a period of time of 10 sec, after a blood sample is arranged on the exposed outer end surface of the electrode device, the electrode system is allowed to reach an equilibrium during which period of time the blood sample further reaches the temperature of the electrode device. After 10 sec, the counter or current electrode is activated and generates a polarizing voltage relative to the reference electrode resulting in that a current peak is generated. By the polarization of the test sample, a current is generated which decays as is indicated by the curves F and G. After 30 sec, the microprocessor starts integrating the areas below the curves F and G, which areas represent the charges transferred to the measuring electrodes in accordance with the equation $\Sigma I \times \Delta t = Q$ and calculates the average charge transferred to the measuring electrodes. During the measuring period, the microprocessor continuously checks whether the responses generated by the measuring electrodes differ from one another. In case a difference between the responses exceeds a predetermined threshold, the microprocessor decides that the measurement is an erroneous measurement and informs the operator thereof on the display and further through an audible alarm generated by the buzzer $X_2$.

It is believed that the measuring period and the integration period, which as shown in Fig. 25 are 60 sec and 30 sec, respectively, in the prototype implementation described above may be reduced to e.g. 30 sec and 15 sec, respectively, by increasing the content of 1,1'-dimethylferrocene. By reducing the measuring period, the time during which the sample is allowed to reach the temperature of the electrode device is obviously also reduced. However, by adapting the measuring routine performed by the microprocessor to carry out an estimation of the decay of the temperature signal on the basis of the variation of the temperature signal detected by the temperature sensor, it is believed that any discrepancy between the temperature detected by the temperature sensor and the temperature of the blood sample may be compensated for.

Fig. 27.    A. The theoretical curves of potential (mV) versus the background (diffusional) current, Id ($\mu$A).
The curves show:
        a: 0.0125 M 1,1'-dimethylferrocene in paraffin oil
        b: 0.025 M 1,1'-dimethylferrocene in paraffin oil
        c: 0.05 M 1,1'-dimethylferrocene in paraffin oil
        d: 0.1 M 1,1'-dimethylferrocene in paraffin oil
    B. The experimental curves of potential (mV) versus $I_d$ ($\mu$A).
The experimental conditions were:
    - temperature: 23°C
    - test medium: phosphate buffer (pH 7.3) with polyvinylpyrrolidone 4.5%
    - electrodes: 4 electrodes embedded in a plastic rod (length - 50 mm).
        2 sensor electrodes diameter 2.5 mm
        1 reference electrode diameter 1.5 mm
        1 counter electrode diameter 1.5 mm

- sensor electrodes: columns of graphite paste ended by stainless steel plugs. Paste composition:

enzyme (6000 IU glucose oxidase/g of carbodiimide-activated graphite particles) covalently linked as described in Example 3

paraffin oil as pasting material (PM)

charge-transfer mediator: 1,1′-dimethylferrocene dissolved in PM.

The curves show:

a: 0.01 M 1,1′-dimethylferrocene in paraffin oil

b: 0.05 M 1,1′-dimethylferrocene in paraffin oil

c: 0.1 M 1,1′-dimethylferrocene in paraffin oil

- reference electrode: column of agarose gel, KCl 1 M ended by a silver/silver chloride plug
- counter electrode: column of agarose gel, KCl 1 M ended by stainless steel plug

From Fig. 27A, it is clear that the $E_{d1/2}$ for the background (diffusional) current is independent of the concentration of the 1,1′-dimethylferrocene solution in paraffin oil presence in the sensor electrode. The $E_{d1/2}$ for Id is approximately 225 mV.

Fig. 28.  A. The theoretical curves of potential (mV) versus background (diffusional) and catalytic current, i.e. $I_d + I_c$ ($\mu$A). The curves simulate different concentrations of glucose:

a 0

b 0.25 $K_m$

c 0.50 $K_m$

d 0.75 $K_m$

e 1.00 $K_m$

f 1.25 $K_m$

where $K_m$ is the Michaelis-Menten constant for the reaction of glucose with glucose oxidase.

B. The experimental curves of potential (mV) versus $I_d + I_c$ ($\mu$A). The experimental conditions were:

- temperature 23°C
- test medium:

curve a: phosphate buffer (pH 7.3) with polyvinylpyrrolidone 4.5%

curve b: human whole blood (89 mg/dl of glucose)

curve c: human whole blood (245 mg/dl of glucose)

- electrodes: 4 electrodes embedded in a 50/50 low density/high density polyethylene rod (length = 50 mm).

2 sensor electrodes, diameter 2.5 mm

1 reference electrode, diameter 1.5 mm

1 counter electrode, diameter 1.5 mm

- working electrodes: columns of paste ended by stainless steel plugs. Paste composition:

enzyme (6000 IU/g of carbodiimide-activated graphite particles) covalently linked as described in Example 3 paraffin oil as pasting material (PM)

charge-transfer mediator: 1,1′-dimethylferrocene 4 x 10$^{-3}$ M dissolved in PM.

- reference electrode: column of agarose gel, KCl 1 M, ended by a silver/silver chloride plug
- counter electrode: column of agarose gel, KCl 1 M ended by stainless steel plug

From Fig. 28A and 28B it is clear that $E_{1/2}$ for ($I_d + I_c$) shifts to approximately 120 mV in the presence of glucose and when the concentration of 1,1′-dimethylferrocene is relatively low corresponding to 4 x 10$^{-3}$ M

Fig. 29  A. The theoretical curves of potential (mV) versus $I_d + I_c$ ($\mu$A). The curves simulate different concentrations of glucose:

a 0

b 0.25 $K_m$

c 0.50 $K_m$

d 0.75 $K_m$

e 1.00 $K_m$

f 1.25 $K_m$

g 1.50 $K_m$

h 1.75 $K_m$

B. The experimental curves of potential (mV) versus $I_d + I_c$ ($\mu$A). The experimental conditions are:

- temperature 23°C
- test medium:

curve a: phosphate buffer (pH 7.3) with polyvinylpyrrolidone 4.5% and 145 meq/l of NaCl

curve b: human whole blood (90 mg/dl of glucose)

curve c: human whole blood (210 mg/dl of glucose)
curve d: human whole blood (347 mg/dl of glucose)
curve e: human whole blood (466 mg/dl of glucose)

- electrodes: 4 electrodes embedded in a 50/50 low density/high density polyethylene rod (length = 50 mm).
2 sensor electrodes, diameter 2.5 mm
1 reference electrode, diameter 1.5 mm
1 counter electrode, diameter 1.5 mm
- sensor electrodes: columns of paste ended by stainless steel plugs. Paste composition:
enzyme (8000 IU/g of carbodiimide-activated graphite particles) covalently linked as described in Example 3 paraffin oil as pasting material (PM)
charge-transfer mediator: 1,1'-dimethylferrocene 0.1 M dissolved in PM.
- reference electrode: silver wire (diameter 100 $\mu$m) coated with silver chloride and fixed in position with polyurethane glue
- counter electrode: silver wire (diameter (100 $\mu$m) fixed in position with polyurethane glue.

From Fig. 29A it is clear that $E_{1/2}$ for the $(I_d+I_c)$ is shifted to about 50 mV due to the higher concentration of 1,1'-dimethylferrocene present in the sensor electrode.

From Fig. 29A it is seen that the operational plateau values are found for potentials of about 100-110 mV when the concentration of 1,1'-dimethylferrocene is about 0.1 M in paraffin oil.

Fig. 30     A.Theoretical curves of potential (mV) versus $I_d+I_c$ ($\mu$A). The curves simulate different concentrations of glucose:

       a 0
       b 0.25 $K_m$
       c 0.50 $K_m$
       d 0.75 $K_m$
       e 1.00 $K_m$
       f 1.25 $K_m$
       g 1.50 $K_m$
       h 1.75 $K_m$

       B. Experimental curves of potential (mV) versus $I_d+I_c$ ($\mu$A). The experimental conditions are the same as given in Fig. 29B but the concentration of 1,1'-dimethylferrocene in paraffin oil was 0.5 M.

Fig. 30     C.Calibration curves for the glucose concentration in human whole blood versus the integrated current ($I_d+I_c$) ($\mu$A sec). The upper curve is obtained using an applied potential of 110 mV, and the lower curve is obtained using an applied potential of 30 mV. The sensor electrode comprises 0.5 M 1,1'-dimethylferrocene in paraffin oil, and the reference electrode is an Ag/AgCl electrode working at 145 mEq/l of chloride ions.

Fig. 30A shows that $E_{1/2}$ for $I_d+I_c$ shifts to about zero or below zero, the concentration of 1,1'-dimethylferrocene in paraffin oil being 0.5 M in the sensor electrode. This point is extremely important since a prerequisite for working at a low potential between the sensor electrode and the reference electrode is that the contribution to the generated current from $I_d$ is very small. To achieve a zero contribution or only a small contribution from $I_d$, the $E_{1/2}$ for the Id should ideally be zero or below zero.

Fig. 30B shows that the operational potentials using 0.5 M 1,1'-dimethylferrocene are low, being about 20-100 mV.

Fig. 30C shows that the calibration curves are linear within the range of 0-350 mg/dl glucose. The curve corresponding to an applied potential of 30 mV has an intercept equal to zero, showing that there is no appreciable interference resulting from the presence of other oxidizable substances in the blood.

**EXAMPLES**

**Example 1**

The prototype implementation of the electronic circuitry of the apparatus according to the present invention described in the legend to figures with reference to Figs. 20-24 was implemented from the following components:

C1     Tantal capacitor SMD; 10$\mu$F/6.3V
C2     Tantal capacitor SMD; 10$\mu$F/6.3V
C3     Ceramic multilayer CAP; 33nF
C4     Ceramic multilayer CAP; 33nF

| | |
|---|---|
| C5 | Tantal capacitor SMD; 4.7μF/6.3V |
| C6 | Tantal capacitor SMD; 4.7μF/6.3V |
| C7 | Ceramic multilayer CAP; 10nF |
| C8 | Ceramic multilayer CAP; 22pF |
| C9 | Ceramic multilayer CAP; 22pF |
| C10 | Tantal capacitor SMD; 1μF/10V |
| D1 | Shottky diode (SMD); BAT54 |
| | LCD Liquid Crystal Display; custom made |
| IC1 | Switched capacitor voltage conv.; LTC1044 |
| IC2 | Micropower voltage reference; LT1004 |
| IC3 | Low-power bifet OP-AMP; AD648 |
| IC4 | Low-power bifet OP-AMP; AD648 |
| IC5 | Low-power bifet OP-AMP; AD648 |
| IC6 | Temperature sensor; S8100 |
| IC7 | Low-power bifet OP-AMP; AD648 |
| IC8 | Single chip microcomputer 4-bit; μPD75328 |
| IC9 | E²PROM; X24LC16 |
| T1 | Transistor; BC849 |
| T2 | Transistor; BC849 |
| L1 | Booster coil |
| R1 | 100kΩ |
| R2 | 100kΩ/1% |
| R3 | 90.1kΩ/1% |
| R4 | 10kΩ/TRIM |
| R5 | 47kΩ/1% |
| R6 | 47kΩ/1% |
| R7 | 10kΩ/TRIM |
| R8 | 100kΩ/1% |
| R9 | 47kΩ/1% |
| R10 | 240kΩ/1% |
| R11 | 240kΩ/1% |
| R12 | 47kΩ/1% |
| R13 | 47Ω/1% |
| R14 | 1MΩ/1% |
| R15 | 1MΩ/1% |
| R16 | 1MΩ/1% |
| R17 | 1MΩ/1% |
| R18 | 200kΩ/TRIM |
| R19 | 1.2MΩ/1% |
| R20 | 10kΩ/TRIM |
| R21 | 39kΩ/1% |
| R22 | 200kΩ/1% |
| R23 | 100kΩ/1% |
| R24 | 100kΩ/1% |
| R25 | 120kΩ/1% |
| R26 | 100kΩ/1% |
| R27 | 100kΩ/1% |
| R28 | 47kΩ/1% |
| R29 | 1MΩ/1% |
| $X_1$ | Resonator Crystal    4.1943MHz |
| $X_2$ | Piezo buzzer |

The above described prototype implementation of the electronic circuitry of the apparatus according to the present invention is to be converted into a custom designed large-scale integrated circuit, in which components and functions identical to or similar to those of the prototype implementation are incorporated. The following features are to be underlined.

The electronic circuit is adapted to carry out the calculation of a measuring result on the basis of the analog measuring signals from a routine in which firstly the temperature detected by the temperature sensor and secondly the specific characteristics of the measuring assembly presently mounted in the apparatus are taken

23

into consideration, which characteristics are input to the microprocessor from the E2PROM IC9 of the block B4C. By the adaptation of the calculation routine to the characteristics of the present electrode device, the electrode device may be manufactured at less critical tolerance ranges, which are compensated for in the specific measuring set-up by the modification of the calculation routine by the characteristics or parameters of the present electrode device and in particular in the present material composition etc. of the measuring electrode material. The electronic circuit is further adapted to continuously measure the temperature in order to determine whether the electrode device is at any time exposed to excessively low or high temperatures beyond a temperature range within which the electrode device is to be permanently maintained in order to ensure that the material of the measuring and sensor electrodes is not damaged due to exposure to low or high temperatures. To this end, the entire electronic circuitry is permanently turned on, however, at a low power consumption mode, in which the temperature sensor and the electronic circuitry connected thereto are permanently supplied with power, while the microprocessor periodically shifts from a low power consumption mode to a normal operational mode, in which the microprocessor checks whether the temperature sensor has generated a signal representing an excessively low or high temperature. In case the temperature sensor and consequently the electrode device have been exposed to too low or too high temperatures, the microprocessor IC8 outputs an alarm to the display block B4B and is turned into a blocking mode in which the microprocessor refuses to carry out a calculation of a measuring result in case the operator attempts to use the apparatus for determining the glucose content of a blood sample.

The microprocessor IC8 autonomously registers when the sample is applied to the sensor electrode, by detecting the presence of the short-circuiting condition between the electrodes of the electrode device, thereby avoiding any problems associated with a need to follow a specified timing sequence.

In the large-scale custom designed integrated implementation of the electronic circuitry, the microprocessor is further connected to an internal storage, such as an additional E2PROM, in which data representing calibration parameters of the individual operation amplifiers, voltage references, temperature sensor, etc. are stored, as well as measurement results.

It has further been realised that a highly accurate temperature sensor may be provided from a crystal oscillator, as the crystal oscillator is a temperature dependent device, which generates a clock frequency dependent on the temperature to which the crystal is exposed. Experiments have confirmed that a highly accurate temperature sensor may be provided from a crystal oscillator, the output signal of which is representative of the temperature to which the crystal is exposed. The above-mentioned additional E2PROM may further include data representing calibration parameters of the temperature sensitive crystal oscillator.

**Example 2**

*Preparation of the graphite paste of the sensor electrode*

1. step: surface oxidation of the graphite powder:
The carbon particles are heated at 100 °C for 40 hours in a continuously rotated flask which is well ventilated by clean, dry atmospheric air,
2. step carbon activation:
42.3 mg of 1-cyclohexyl-3-(2-morpholinoethyl)carbodiimide-metho-p-toluenesulfonate (N-cyclohexyl-N′-β-[N-methylmorpholino)ethyl]carbodiimide p-toluenesulfonate salt) is dissolved in 3 ml of acetic acid/acetate buffer of pH 4.76. The solution is mixed with 1 gram of oxidized graphite particles from step 1. The mixture is continuously stirred at room temperature for 2 hours. Then the mixture is washed 7 times with distilled water and dried by evaporation of the solvent at room temperature or by lyophilization.
3. step enzyme immobilization:
8000 IU glucose oxidase per gram activated graphite is dissolved in 2 ml phosphate buffer pH 7.3 per gram carbodiimide-activated graphite. The mixture is continuously stirred at 4 °C for 16 hours. Then the mixture is dried by evaporation of the solvent at room temperature or by lyophilization. The dry powder is sieved through a 48 mesh nylon sieve.
4. step
0.6 ml of paraffin oil is mixed with 4-5 ml of petroleum ether or n-pentane per gram activated graphite. 12.8 mg 1,1′-dimethylferrocene is dissolved in this mixture.
5. step
The activated graphite particles comprising the immobilized enzyme from step 3 is mixed with the mixture from step 4 followed by evaporation at room temperature of the petroleum ether or the n-pentane.
6. step filling of the channels in the electrode body:
The mixture from step 5 is filled into the channel(s) of the sensor electrode in vacuo.

## Example 3

Preparation of the sensor electrode, the reference electrode and the counter electrode and the filling of the electrodes into the electrode device body

In the following is described the preparation of the electrodes of an electrode device comprising four electrodes, each filled into a channel of the electrode device body. The electrodes are two sensor electrodes, a reference electrode and a counter electrode.

*Counter electrode and reference electrode*

The counter electrode and the reference electrode channels are filled with 0.2 mm of silver wire and 0.2 mm of silver wire coated with a layer of silver chloride, respectively. The coating of the silver wire with silver chloride to obtain the reference electrode is done by placing the silver wire in a 1 % w/w solution of ferric chloride (Merck art. 3943) in a 1 M solution of hydrogen chloride (Merck art. 317) for about 30 min. Then the silver wire coated with silver chloride (reference electrode) and the silver wire (counter electrode) are fixed in a plug of brass in the bottom of the reference electrode channel and the counter electrode channel, respectively, in the electrode device body and the fixation in the channels is done by application of a two-component epoxy adhesive. The application is done using a 5 ml syringe and a 18G needle and precautions are made to remove any gas present in the channel. The two-component epoxy adhesive is prepared substantially immediately before use by mixing 4 parts of glue with 1 part of hardener.

After one day, the two-component epoxy adhesive has stiffened and the electrode device body comprising the counter electrode and the reference electrode can be filled with the sensor electrode material.

*Graphite-containing paste*

*Oxidation of the graphite particles*

A suitable amount of graphite particles (Fluka art. 50870) are weighed and charged into a flask which is placed in a Rotavapor. The graphite particles are oxidized by means of dry atmospheric air for at least 48 hours, the air being substituted with 99.9 % pure atmospheric air under pressure. The oxidation takes place at 100 °C by means of a termostated bath of silicone oil. In order to ensure an optimal movement in the graphite particles, a few small bullets of teflon is added to the flask containing the graphite particles.

*Carbodiimide activation*

The carbodiimide (1-cyclohexyl-3-(2-morpholinoethyl)carbodiimide-metho-p-toluenesulfonate) (Sigma art. C-1011) is used in an amount corresponding to 0.1 mol per gram surface oxidized graphite particles, the amount being equivalent to 42.36 mg carbodiimide per gram surface oxidized graphite particles.
The appropriate amount is dissolved in 2-3 ml of 0.05 M acetate buffer per gram oxidized graphite.
The acetate buffer is prepared from 2.05 g sodium acetate (Merck art. 6268) and 1.50 g = 1.43 ml glacial acetic acid (Merck art. 63) per liter buffer. pH is adjusted to 4.76 by addition of sodium hydroxide or acetic acid.
The volume to dissolve the carbodiimide is not critical, but the pH has to be about 4.76 in order to achieve a correct coupling of the carbodiimide to the graphite particles.
The dissolved carbodiimide is added to the oxidized graphite particles and the mixture is continuously rotated on Rotavapor for about 2 hours at room temperature. Then the excess of carbodiimide is removed by centrifugation of the mixture for 5 min at 1500 rpm and subsequently the supernatant is discarded. The residue is washed with distilled water and the mixture is re-centrifuged. The latter step is repeated until pH in the washings has increased from 4.76 to 5-6.
The oxidized and carbodiimide-activated graphite particles prepared in the above-mentioned way, is air dried by means of a ventilator and when the powder is dry, the graphite particles can be loaded with the enzyme. An appropriate drying of the particles is controlled by two subsequent weighings with an interval of 10 min. Substantially equal results indicate that the particles are dry.
The carbodiimide-activated graphite particles can not be stored but must be used shortly after the preparation.

*Immobilization of enzyme on the graphite particles*

In the following all reagents, solutions and utensils should have a temperature of 4 °C to avoid any denaturation of the enzyme or loss of activity during the preparation.

Glucose oxidase (type IV, Sigma art. G-2133) is used in an amount corresponding to 8000 IU/g carbodiimide-activated graphite particles. The appropriate amount is dissolved in 1 ml 0.05 M phosphate buffer solution and 0.1 ml 4% w/w aqueous solution of glutaraldehyde is added per gram carbodiimide-activated graphite particles.

The phosphate buffer is made of 4.45 g $Na_2HPO_4$ (Merck art. 6580) and 3.45 g $NaH_2PO_4$ (Merck art. 6346) per liter buffer solution. pH is adjusted to 7.30.

The 4% w/w solution of glutaraldehyde is made by diluting a 25 % w/w solution (Serva art. 23114) with distilled water.

The dried carbodiimide-activated graphite particles are weighed and charged into a flask and then the solution containing the enzyme is added. After mixing at 4 °C for at least 16 hours, the graphite particles comprising the glucose oxidase (GOD-containing graphite particles) are air-dried.

The GOD-containing graphite particles obtained can be stored at 4 °C.

*Pasting material containing the charge-transfer mediator*

Finally, the GOD-containing graphite particles is carefully mixed with paraffin oil containing the 1,1'-dimethylferrocene.

The paraffin oil (Merck art. 7161) in an amount corresponding to 0.6 ml = 0.53 g per gram GOD-containing graphite is added to 1,1'-dimethylferrocene (Stream Chemical Inc. art. 26-1500) in an amount corresponding to 12.86 mg per gram GOD-containing graphite.

The 1,1'-dimethylferrocene is dissolved in the paraffin oil and is mixed with the GOD-containing graphite particles. An appropriate amount of n-pentane is added in order to facilitate the intimate mixing of the components and to ensure that the solution of 1,1'-dimethylferrocene in paraffin oil is uniformly distributed in the GOD-containing graphite particles.

After mixing, the paste is left for one day to allow the n-pentane to evaporate and at suitable intervals stirred to facilitate the evaporation, and then the paste is ready to be filled into the electrode device, e.g. in vacuo.

## Example 4

*Preparation of a sensor electrode comprising the glucose oxidase in an inactive form*

The preparation of sensor electrode wherein the glucose oxidase is present in an inactivated form is performed as described above, but the glucose oxidase is after immobilization on the graphite particles inactivated by exposing the GOD-containing graphite to 50°C for 24 hours.

## Example 5

*Preparation of the sensor electrode and filling the sensor electrode into a channel of the electrode device body*

The GOD-containing particles were prepared as described in Example 2.

The GOD-containing graphite is mixed with a sufficient amount of the non-polar pasting material, e.g. paraffin oil, in which the charge transfer mediator, e.g. 1,1'-dimethylferrocene has been dissolved. Preferably, the 1,1'-dimethylferrocene solution is about 0.1 M in paraffin oil. The amount of the approximately 0.1 M 1,1'-dimethylferrocene solution in paraffin oil is larger then the required and final amount in the sensor electrode. The reason therefore is that it should be possible to obtain a dispersion of GOD-containing graphite in the 1,1'-dimethylferrocene solution, which dispersion has a suitable consistency to allow the channel(s) in the electrode body to be filled. The excessive amount of the 1,1'-dimethylferrocene solution is easily removed after filling of the channel(s) of the sensor electrode in the electrode body by centrifugation. The amount used of the 1,1'-dimethylferrocene is preferably 3-4 times greater than the necessary amount to establish a final concentration of about 10-15 mg 1,1'-dimethylferrocene per gram GOD-containing graphite in the sensor electrode, and an amount of about 2-3 ml 1,1'-dimethylferrocene solution is normally used per gram GOD-containing graphite.

To fill the sensor electrode channels(s), the electrode device body is placed in a special holder having a small funnel-shaped reservoir at the top. The dispersion containing the GOD-containing graphite and the 1,1'-dimethylferrocene in paraffin oil solution is then poured into the reservoir. The electrode device body equipped with the filled reservoir is then centrifuged at about 15,000 - 50,000 x *g* for about 5-30 min, the centrifugation preferably being performed at about 20,000 x *g* for about 10 min, the electrode body device being positioned in an inverted position during centrifugation (i.e. that end of the device from which slices later are removed being uppermost). By the centrifugation the air is displaced from the channels and the GOD-containing graphite particles sediment, the interstices between the particles being filled with the 1,1'-dimethylferrocene in paraffin oil and the excess amount of 1,1'-dimethylferrocene in paraffin oil remaining as a supernatant in the upper part. After centrifugation the excess 1,1'-dimethylferrocene/paraffin oil is easily removed and the surface of the sensor electrode is levelled with the surface of the electrode device body by cutting off a suitable cross-sectional portion of the electrode device.

**Example 6**

*Preparation of a reference electrode comprising an Ag/AgCl electrode*

At the bottom of the reference electrode channel of the electrode device body is placed an Ag/AgCl reference electrode and the channel is filled with an agarose gel containing a 1 M solution of KCl.

Alternatively, the channel may be filled with a plastic embedding a powder of fine silver and silver chloride particles.

**Example 7**

*Measuring technique*

In the following is given an example of the measuring technique. The measurement is performed with an apparatus according to the invention having a sensor electrode comprising 0.1 M 1,1'-dimethylferrocene in paraffin oil and a reference electrode of Ag/AgCl in an agarose gel containing 1.0 M KCl.

After having cut off a slice of the electrode device, a sample of the aqueous medium is applied on the fresh surface of the electrode device. The sample is preferably a sample of whole blood.

After 10-15 seconds, a constant potential (corresponding to 160 mV when a Ag/AgCl reference electrode comprising 1.0 M KCl is used) is applied. After further 10-20 seconds, the currents are then integrated over 30 seconds. The charges measured are proportional to the concentration of the glucose in the sample.

Using 0.5 M 1,1'-dimethylferrocene in paraffin oil, the measuring condition can be changed. Thus, the application of a constant potential (corresponding to 30 mV when a Ag/AgCl reference electrode working at a chloride ion concentration of 145 meq/l) is done substantially immediately. After about 1 second, the current is integrated over about 5 seconds. The charges measured are proportional to the concentration of the glucose in the sample.

Final result takes into account the known background (diffusion) current and a temperature coefficient of 4.5% per degree celsius.

**Claims**

1. A method of voltametric detection of at least one constituent of a fluid sample of animal or human whole blood by means of a measuring apparatus containing an integrated electrode device including at least one enzyme sensor electrode and a reference electrode having an exposed surface part at a free end portion of the integrated electrode device and extending longitudinally through said end portion, said method being characterized in that there as reference electrode is used an Ag/AgCl electrode working at a chloride ion concentration of about 145 meq/l and that before applying the sample on the exposed surface of the electrode device, said electrode device is moved longitudinally outward by an increment and subsequently the outer end section of said free end portion of the integrated electrode device is removed by means of removing means mounted movably on the measuring apparatus, so as to provide a new exposed surface part of the integrated electrode device, and that subsequently said new exposed surface part of the integrated electrode device is exposed to said fluid sample, so as to generate a measuring signal representative of said constituent of the sample.

2. A method according to claim 1, wherein the outer end section of the free end portion of the electrode device is removed by cutting.

3. A method according to any of the preceding claims, characterized in that said constituent is glucose.

4. A method according to any of the preceding claims, characterized in that there is used an enzyme sensor electrode in the form of a paste comprising electrically conductive particles and a pasting material, an oxidase enzyme and a charge-transfer mediator, and in that the charge-transfer mediator in its reduced form is dissolved in the pasting material in a concentration which is at least sufficient to ensure that the concentration of the charge-transfer mediator in its oxidized form is substantially constant at the exposed surface of the sensor electrode during the measurement, the charge-transfer mediator in its reduced form optionally further being present in undissolved form in the paste.

5. A method according to claim 4, characterized in that there as said paste is used a paste comprising graphit particles and a non-polar pasting material.

6. A method according to claim 4 or 5, characterized in that the concentration of the reduced form of the charge-transfer mediator in the pasting material is from 0.01 to 1.5 M, preferably from 0.05 to 1.0 M, more preferred from 0.07 to 0.7 M, and most preferred from 0.4 to 0.7 M.

7. A method according to any of claims 5 to 6, characterized in that there as charge-transfer mediator is used an organo-metallic compound, the reduced form of which is soluble in a non-polar pasting material.

8. A method according to claim 7, characterized in that the organometallic compound is a metallocene or a derivative thereof, preferably a ferrocene derivative.

9. A method according to claim 8, characterized in that the ferrocene derivative is 1,1′-dimethylferrocene.

10. A method according to any of claims 5 to 9, characterized in that there as non-polar pasting material is used paraffin oil.

11. A method according to any of the preceding claims 1 to 10, characterized in that the measurement is performed at a potential between the sensor electrode and the Ag/AgCl reference electrode in communication with the fluid sample in a range of from 0 to 250 mV.

12. A method according to claim 11, characterized in that the measurement is performed at a potential between the sensor electrode and the Ag/AgCl reference electrode in a range of from 0 to 200 mV, preferably from 10 to 150 mV, and in particular from 20 to 120 mV.

13. A method according to claim 12, characterized in that the measurement is performed at a potential of about 110 mV.

14. A method according to claim 12, characterized in that the measurement is performed at a potential of about 50 mV.

15. A method according to claim 12, characterized in that the measurement is performed at a potential of about 30 mV.

16. An electrode device for use in the method according to any of the preceding claims 1 to 15 comprising an electrode body member (16) having a free end portion (21), at least one sensor electrode (18) extending axially through said end portion and having a substantially uniform cross-sectional area within said end portion, and a reference electrode (19) also extending axially through the free end portion (21) of the electrode body member, each electrode being received in a bore extending longitudinally through the electrode body member and having an exposed surface part at the free end portion of the electrode body member, characterized in that the reference electrode is an Ag/AgCl electrode and that the sensor electrode is an electrically conductive electrode comprising a paste of electrically conductive particles and a pasting material containing an oxidase enzyme and a charge-transfer mediator, being uniformly distributed in the paste and in its reduced form being dissolved in the pasting material in a concentration which is at least sufficient to ensure that the concentration of the charge-transfer mediator in its oxidized form is substantially constant at the exposed surface of the sensor electrode during the measurement, the charge-transfer mediator in its reduced form optionally further being present in undissolved form in the paste.

17. An electrode device according to claim 16, characterized in that it further contains a counterelectrode (20) extending axially through the free end portion (21) of the electrode body member (16).

18. An electrode device according to claim 16, characterized in that the paste comprises graphite particles and a non-polar pasting material which is substantially immiscible with water and which is capable of dissolving the charge-transfer mediator in its reduced form.

19. An electrode device according to any of the preceding claims 16 to 18, characterized in that the oxidase enzyme is glucose oxidase which is bonded and/or adsorbed to the electrically conductive particles.

20. An electrode device according to any of the preceding claims 16 to 19, characterized in that the concentration of the reduced form of the charge-transfer mediator in the pasting material is from 0.01 to 1.5 M, preferably from 0.05 to 1.0 M, more preferably from 0.07 to 0.7 M, and most preferred from 0.4 to 0.7 M.

21. An electrode device according to any of the preceding claims 16 to 20, characterized in that the non-polar pasting material is paraffin oil.

22. An electrode device according to any of the preceding claims 16 to 21, characterized in that the electrode body member is made from a polymeric material.

23. An electrode device according to claim 22, characterized in that the polymeric material is a mixture of high density polyethylene and low density polyethylene, the weight ratio between the amount of high density and low density polyethylene preferably being about 1.

24. An electrode device according to any of preceding claims 16 to 23, characterized in that it comprises two sensor electrodes (18), an Ag/AgCl reference electrode (19) and a counter electrode (20).

25. An electrode device according to claim 24, characterized in that the two sensor electrodes are of the same type.

26. An electrode device according to claim 25, characterized in that the two sensor electrodes are different, one electrode comprising glucose oxidase in a catalytically active form, the other electrode comprising glucose oxidase in an inactivated form.

27. A measuring apparatus for performing the method according to claim 1-15 to measure at least one constituent of a fluid sample of animal or human whole blood, using an electrode according any one of the claims 16-26, characterized in that it comprises

a body member (10) defining an outwardly open cavity (14) for receiving the electrode device (15) therein,

removing means (27) mounted on the body member (10) so as to be movable in relation thereto along a path intersecting said cavity (14) for removing an outer end section from a free end portion of the electrode device (15) received in the cavity (14),

means (30-34 and 37) for moving the electrode device outwardly by an increment each time the removing means has been operated,

an electronic measuring circuitry (35) for processing measuring signals received from the electrode or electrodes of the electrode device, and

connecting means for electrically connecting the electrode or electrodes of the electrode device to the electronic measuring circuitry when the electrode device is received in said cavity.

28. An apparatus according to claim 27, characterized in that said removing means define a cutting edge (28) for cutting the outer end section from the free end portion of the electrode device when the removing means are moved along said path.

29. An apparatus according to claim 27 or 28, characterized in that the removing means are mounted so as to be movable reciprocatingly along said path.

30. An apparatus according to any of the claims 27 to 29, characterized in that the removing means are mounted on the apparatus body member so as to be rotatable in relation thereto.

31. An apparatus according claim 30, characterized in that it further comprises means for permitting rotation of the removing means in one direction only.

32. An apparatus according to claim 31, characterized in that said rotation permitting means comprise a ratchet mechanism.

**33.** An apparatus according to any of claims 27 to 32, characterized in that said moving means comprise a driving member driven by the movement of said removing means.

**34.** An apparatus according to any of claims 27 to 33, characterized in that the removing means are mounted on a lid-like member (11) which is movable to a closing, non-operative position.

**35.** An apparatus according to any of claims 27 to 34, characterized in that it further comprises
a skin-puncturing member (46) mounted in the apparatus body member (10) so as to be movable between a retracted position and an extended position,
biassing means (52, 53) for biassing the puncturing member towards its extended position, and
releasable locking means (54) for retaining the puncturing member in its retracted position against the bias of the biassing means, whereby the puncturing member may perform a sudden skin-puncturing movement from its retracted to its extended position, when the locking means are released.

**36.** An apparatus according to any of claims 16 to 35, characterized in that it further comprises a visual display (36) for displaying the result of measurements performed by the electrode or electrodes of the electrode device.

**Patentansprüche**

**1.** Verfahren zum voltametrischen Erfassen wenigstens eines Bestandteiles einer Fluidprobe tierischen oder menschlichen Gesamtblutes mittels eines Meßgerätes, das eine integrierte Elektrodenvorrichtung enthält, welche wenigstens eine Enzym-Sensorelektrode und eine Bezugselektrode umfaßt, die einen freiliegenden Oberflächenteil an einem freien Endabschnitt der integrierten Elektrodenvorrichtung haben und sich in Längsrichtung durch den Endabschnitt erstrecken, wobei das Verfahren dadurch gekennzeichnet ist, daß dabei als Bezugselektrode eine Ag/AgCl-Elektrode verwendet wird, die mit einer Chloridionenkonzentration von etwa 145 meq/l arbeitet, und daß vor dem Aufgeben der Probe auf die freiliegende Oberfläche der Elektrodenvorrichtung die Elektrodenvorrichtung in Längsrichtung nach außen um einen Betrag bewegt wird und nachfolgend der äußere Endbereich des freien Endabschnittes der integrierten Elektrodenvorrichtung mittels einer Entferneinrichtung entfernt wird, die bewegbar auf dem Meßgerät angebracht ist, um so einen neuen freiliegenden Oberflächenteil der integrierten Elektrodenvorrichtung zu schaffen, und daß nachfolgend der neue freiliegende Oberflächenteil der integrierten Elektrodenvorrichtung der Flüssigkeitsprobe ausgesetzt wird, um so ein Meßsignal zu erzeugen, das für den Bestandteil der Probe repräsentativ ist.

**2.** Verfahren nach Anspruch 1, bei dem der äußere Endbereich des freien Endabschnittes der Elektrodenvorrichtung durch Schneiden entfernt wird.

**3.** Verfahren nach einem der vorangehenden Ansprüche, dadurch gekennzeichnet, daß der Bestandteil Glukose ist.

**4.** Verfahren nach einem der vorangehenden Ansprüche, dadurch gekennzeichnet, daß dabei eine Enzym-Sensorelektrode in der Form einer Paste, die elektrisch leitende Teilchen und ein Klebermaterial, ein Oxidaseenzym und einen Ladungs-Übertragungs-Vermittler aufweist, benutzt wird, und daß der Ladungs-Übertragungs-Vermittler in seiner reduzierten Form in dem Klebermaterial in einer Konzentration gelöst ist, die wenigstens ausreichend ist, um sicherzustellen, daß die Konzentration des Ladungs-Übertragungs-Vermittlers in seiner oxidierten Form an der freiliegenden Fläche der Sensorelektrode während der Messung im wesentlichen konstant ist, wobei der Ladungs-Übertragungs-Vermittler in seiner reduzierten Form optional weiter in ungelöster Form in der Paste vorliegt.

**5.** Verfahren nach Anspruch 4, dadurch gekennzeichnet, daß als die Paste eine Paste verwendet wird, die Graphitteilchen und ein nicht polares Klebermaterial aufweist.

**6.** Verfahren nach Anspruch 4 oder 5, dadurch gekennzeichnet, daß die Konzentration der reduzierten Form des Ladungs-Übertragungs-Vermittlers in dem Klebermaterial zwischen 0,01 bis 1,5 M, bevorzugt zwischen 0,05 bis 1,0 M, weiter bevorzugt zwischen 0,07 bis 0,7 M und am meisten bevorzugt von 0,04 bis 0,7 M liegt.

7. Verfahren nach einem der Ansprüche 5 bis 6, dadurch gekennzeichnet, daß als Ladungs-Übertragungs-Vermittler eine organometallische Verbindung benutzt wird, deren reduzierte Form in einem nicht polaren Klebermaterial löslich ist.

8. Verfahren nach Anspruch 7, dadurch gekennzeichnet, daß die organometallische Verbindung ein Metallocen oder ein Derivat davon ist, bevorzugt ein Ferrocen-Derivat.

9. Verfahren nach Anspruch 8, dadurch gekennzeichnet, daß das Ferrocen-Derivat 1,1'-Dimethylferrocen ist.

10. Verfahren nach einem der Ansprüche 5 bis 9, dadurch gekennzeichnet, daß als nicht polares Klebermaterial Paraffinöl benutzt wird.

11. Verfahren nach einem der vorangehenden Ansprüche 1 bis 10, dadurch gekennzeichnet, daß die Messung mit einem Potential zwischen der Sensorelektrode und der Ag/AgCl-Bezugselektrode in Kommunikation mit der Fluidprobe in einem Bereich von 0 bis 250 mV durchgeführt wird.

12. Verfahren nach Anspruch 11, dadurch gekennzeichnet, daß die Messung mit einem Potential zwischen der Sensorelektrode und der Ag/AgCl-Bezugselektrode in einem Bereich von 0 bis 200 mV, bevorzugt von 10 bis 150 mV und insbesondere von 20 bis 120 mV durchgeführt wird.

13. Verfahren nach Anspruch 12, dadurch gekennzeichnet, daß die Messung mit einem Potential von etwa 110 mV durchgeführt wird.

14. Verfahren nach Anspruch 12, dadurch gekennzeichnet, daß die Messung mit einem Potential von etwa 50 mV durchgeführt wird.

15. Verfahren nach Anspruch 12, dadurch gekennzeichnet, daß die Messung mit einem Potential von etwa 30 mV durchgeführt wird.

16. Elektrodenvorrichtung zur Verwendung in dem Verfahren nach einem der vorangehenden Ansprüche 1 bis 15, mit einem Elektrodenkörperelement (16), das einen freien Endabschnitt (21), wenigstens eine Sensorelektrode (18), die sich axial durch den Endabschnitt erstreckt und einen im wesentlichen gleichförmigen Querschnittsbereich innerhalb des Endabschnittes hat, und eine Bezugselektrode (19), die sich auch axial durch den freien Endabschnitt (21) des Elektrodenkörperelementes erstreckt, hat, wobei jede Elektrode in einer Bohrung aufgenommen ist, die sich in Längsrichtung durch das Elektrodenkörperelement erstreckt und einen freiliegenden Oberflächenteil an dem freien Endabschnitt des Elektrodenkörperele mentes hat, dadurch gekennzeichnet, daß die Bezugselektrode eine Ag/AgCl-Elektrode ist und daß die Sensorelektrode eine elektrisch leitende Elektrode ist, welche eine Paste elektrisch leitender Teilchen und ein Klebermaterial aufweist, das ein Oxidaseenzym und einen Ladungs-Übertragungs-Vermittler, welcher in der Paste gleichförmig verteilt ist und in seiner reduzierten Form in dem Klebermaterial in einer Konzentration gelöst ist, die wenigstens ausreichend ist, um sicherzustellen, daß die Konzentration des Ladungs-Übertragungs-Vermittlers in seiner oxidierten Form an der freiliegenden Fläche der Sensorelektrode während der Messung im wesentlichen konstant ist, enthält, wobei der Ladungs-Übertragungs-Vermittler in seiner reduzierten Form optional weiter in ungelöster Form in der Paste vorliegt.

17. Elektrodenvorrichtung nach Anspruch 16, dadurch gekennzeichnet, daß sie weiter eine Gegenelektrode (20) enthält, die sich axial durch den freien Endabschnitt (21) des Elektrodenkörperelementes (16) erstreckt.

18. Elektrodenvorrichtung nach Anspruch 16, dadurch gekennzeichnet, daß die Paste Graphitteilchen und ein nicht polares Klebermaterial aufweist, das im wesentlichen mit Wasser unmischbar ist und das in der Lage ist, den Ladungs-Übertragungs-Vermittler in seiner reduzierten Form zu lösen.

19. Elektrodenvorrichtung nach einem der vorangehenden Ansprüche 16 bis 18, dadurch gekennzeichnet, daß das Oxidase-enzym Glukoseoxidase ist, das an die elektrisch leitenden Teilchen gebunden und/oder adsorbiert ist.

20. Elektrodenvorrichtung nach einem der vorangehenden Ansprüche 16 bis 19, dadurch gekennzeichnet, daß die Konzentration der reduzierten Form des Ladungs-Übertragungs-Vermittlers in dem Klebermaterial zwischen 0,01 bis 1,5 M, bevorzugt zwischen 0,05 bis 1,0 M, weiter bevorzugt zwischen 0,07 bis 0,7 M und am meisten bevorzugt zwischen 0,4 bis 0,7 M liegt.

21. Elektrodenvorrichtung nach einem der vorangehenden Ansprüche 16 bis 20, dadurch gekennzeichnet, daß das nicht polare Klebermaterial Paraffinöl ist.

22. Elektrodenvorrichtung nach einem der vorangehenden Ansprüche 16 bis 21, dadurch gekennzeichnet, daß das Elektrodenkörperelement aus einem polymeren Material hergestellt ist.

23. Elektrodenvorrichtung nach Anspruch 22, dadurch gekennzeichnet, daß das polymere Material eine Mischung aus hochdichtem Polyethylen und niedrigdichtem Polyethylen ist, wobei das Gewichtsverhältnis zwischen der Menge an hochdichtem und niedrigdichtem Polyethylen bevorzugt ungefähr 1 ist.

24. Elektrodenvorrichtung nach einem der vorangehenden Ansprüche 16 bis 23, dadurch gekennzeichnet, daß sie zwei Sensorelektroden (18), eine Ag/AgCl-Bezugselektrode (19) und eine Gegenelektrode (20) aufweist.

25. Elektrodenvorrichtung nach Anspruch 24, dadurch gekennzeichnet, daß die beiden Sensorelektroden vom selben Typ sind.

26. Elektrodenvorrichtung nach Anspruch 25, dadurch gekennzeichnet, daß die beiden Sensorelektroden unterschiedlich sind, wobei eine Elektrode Glukoseoxidase in einer katalytisch aktiven Form aufweist, die andere Elektrode Glukoseoxidase in einer katalytisch inaktiven Form aufweist.

27. Meßgerät zum Durchführen des Verfahrens nach Anspruch 1 - 15 zum Messen wenigstens eines Bestandteiles einer Fluidprobe tierischen oder menschlichen Gesamtbluts, wobei eine Elektrode nach einem der Ansprüche 16 - 26 verwendet wird, dadurch gekennzeichnet, daß es aufweist

ein Körperelement (10), das einen nach außen offenen Hohlraum (14) zum Aufnehmen der Elektrodenvorrichtung (15) darin definiert,

eine Entferneinrichtung (27) die auf dem Körperelement (10) so angebracht ist, daß sie in Bezug darauf entlang eines Weges bewegbar ist, der den Hohlraum (14) überschneidet, zum Entfernen eines äußeren Endbereiches von einem freien Endabschnitt der Elektrodenvorrichtung (15), die in dem Hohlraum (14) aufgenommen ist,

eine Einrichtung (30 - 34 und 37) zum Bewegen der Elektrodenvorrichtung um einen Betrag, jedesmal wenn die Entferneinrichtung betätigt worden ist, nach außen,

eine elektronische Meßschaltung (35) zum Verarbeiten von Meßsignalen, die von der Elektrode oder den Elektroden der Elektrodenvorrichtung erhalten worden sind, und

Verbindungsmittel zum elektrischen Verbinden der Elektrode oder Elektroden der Elektrodenvorrichtung mit der elektronischen Meßschaltung, wenn die Elektrodenvorrichtung in dem Hohlraum aufgenommen ist.

28. Gerät nach Anspruch 27, dadurch gekennzeichnet, daß die Entferneinrichtung eine Schneidkante (28) zum Abschneiden des äußeren Endbereiches von dem freien Endabschnitt der Elektrodenvorrichtung, wenn die Entferneinrichtung entlang des Weges bewegt wird, definiert.

29. Gerät nach Anspruch 27 oder 28, dadurch gekennzeichnet, daß die Entferneinrichtung so angebracht ist, daß sie entlang des Weges hin- und herbewegbar ist.

30. Gerät nach einem der Ansprüche 27 bis 29, dadurch gekennzeichnet, daß die Entferneinrichtung auf dem Gerätekörperelement so angebracht ist, daß sie in Bezug dazu drehbar ist.

31. Gerät nach Anspruch 30, dadurch gekennzeichnet, daß es weiter Mittel zum Ermöglichen der Drehung der Entferneinrichtung nur in eine Richtung aufweist.

32. Gerät nach Anspruch 31, dadurch gekennzeichnet, daß die die Drehung erlaubenden Mittel einen Sperrklinkenmechanismus aufweisen.

33. Gerät nach einem der Ansprüche 27 bis 32, dadurch gekennzeichnet, daß die Bewegeungseinrichtung ein Antriebselement aufweist, das von der Bewegung der Entferneinrichtung angetrieben wird.

**34.** Gerät nach einem der Ansprüche 27 bis 33, dadurch gekennzeichnet, daß die Entferneinrichtung auf einem deckelähnlichen Element (11) angebracht ist, der in eine schließende, nicht-operative Position bewegbar ist.

**35.** Gerät nach einem der Ansprüche 27 bis 34, dadurch gekennzeichnet, daß es weiter aufweist
ein Hautpunktionselement (46), das in dem Gerätekörperelement (10) so angebracht ist, daß es zwischen einer zurückgezogenen Position und einer vorgeschobenen Position bewegbar ist,
eine Vorspanneinrichtung (52, 53) zum Vorspannen des Punktionselementes in Richtung auf seine vorgeschobene Position, und
eine freigebbare Verriegelungseinrichtung (54) zum Zurückhalten des Punktionselementes in seienr zurückgezogenen Position gegen die Vorspennung der Vorspanneinrichtung, wodurch das Punktionselement eine plötzliche, die Haut punktierende Bewegung aus seiner zurückgezogenen in seine vorgeschobene Position durchführen kann, wenn die Verriegelungseinrichtung freigegeben wird.

**36.** Gerät nach einem der Ansprüche 16 bis 35, dadurch gekennzeichnet, daß es weiterhin eine visuelle Anzeiqe zum Anzeigen des Ergebnisses von Messungen aufweist, die von der Elektrode oder den Elektroden der Elektrodenvorrichtung vollführt worden sind.

## Revendications

**1.** Méthode de détection voltamétrique d'au moins un constituant d'un échantillon liquide de sang total animal ou humain à l'aide d'un appareil de mesure contenant un système d'électrodes intégré comprenant au moins une électrode détectrice enzymatique et une électrode de référence, ayant une partie de la surface exposée au niveau d'une portion terminale libre du système d'électrodes intégré, et s'étendant dans le sens de la longueur à travers ladite portion terminale, ladite méthode étant caractérisée en ce que l'on utilise comme électrode de référence une électrode Ag/AgCl fonctionnant à une concentration d'ions chlorure d'environ 145 méq/l et en ce que, avant d'appliquer l'échantillon sur la surface exposée du système d'électrodes, on fait avancer ledit système d'électrodes d'un incrément vers l'extérieur dans le sens de la longueur, puis on enlève la section terminale externe de ladite portion terminale libre du système d'électrodes intégré à l'aide d'un moyen d'enlèvement monté de façon mobile sur l'appareil de mesure, de façon à fournir une nouvelle partie de surface exposée du système d'électrodes intégré, et en ce qu'ensuite on expose ladite nouvelle partie de surface exposée du système d'électrodes intégré audit échantillon liquide, de façon à produire un signal de mesure représentatif dudit constituant de l'échantillon.

**2.** Méthode selon la revendication 1, dans laquelle la section terminale externe de la portion terminale libre du système d'électrodes est enlevée par coupure.

**3.** Méthode selon l'une quelconque des revendications précédentes, caractérisée en ce que ledit constituant est le glucose.

**4.** Méthode selon l'une quelconque des revendications précédentes, caractérisée en ce que l'on utilise une électrode détectrice enzymatique sous forme d'une pâte comprenant des particules conductrices de l'électricité et une matière formant une pâte, une enzyme oxydase et un médiateur de transfert de charge, et en ce que le médiateur de transfert de charge sous forme réduite est dissous dans la matière formant une pâte en une concentration qui est au moins suffisante pour assurer pendant la mesure une concentration essentiellement constante du médiateur de transfert de charge sous forme oxydée au niveau de la surface exposée de l'électrode détectrice, le médiateur de transfert de charge sous forme réduite étant éventuellement présent en plus sous forme non dissoute dans la pâte.

**5.** Méthode selon la revendication 4, caractérisée en ce que l'on utilise comme pâte une pâte comprenant des particules de graphite et une matière formant une pâte non polaire.

**6.** Méthode selon la revendication 4 ou 5, caractérisée en ce que la concentration de la forme réduite du médiateur de transfert de charge dans la matière formant une pâte est de 0,01 à 1,5 M, de préférence de 0,05 à 1,0 M, de façon davantage préférée de 0,07 à 0,7 M, et de la façon la plus préférée de 0,4 à 0,7 M.

**7.** Méthode selon l'une quelconque des revendications 5 à 6, caractérisée en ce que l'on utilise comme mé-

EP 0 478 550 B1

diateur de transfert de charge un composé organométallique dont la forme réduite est soluble dans une matière non polaire formant une pâte.

8. Méthode selon la revendication 7, caractérisée en ce que le composé organométallique est un métallocène ou un de ses dérivés, de préférence un dérivé du ferrocène.

9. Méthode selon la revendication 8, caractérisée en ce que le dérivé de ferrocène est le 1,1′-diméthylferrocène.

10. Méthode selon l'une quelconque des revendications 5 à 9, caractérisée en ce que l'on utilise de l'huile de paraffine comme matière non polaire formant une pâte.

11. Méthode selon l'une quelconque des revendications 1 à 10 précédentes, caractérisée en ce que l'on effectue la mesure à un potentiel entre l'électrode détectrice et l'électrode de référence Ag/AgCl en communication avec l'échantillon liquide compris entre 0 et 250 mV.

12. Méthode selon la revendication 11, caractérisée en ce que l'on effectue la mesure à un potentiel entre l'électrode détectrice et l'électrode de référence Ag/AgCl compris entre 0 et 200 mV, de préférence entre 10 et 150 mV, et en particulier entre 20 et 120 mV.

13. Méthode selon la revendication 12, caractérisée en ce que l'on effectue la mesure à un potentiel d'environ 110 mV.

14. Méthode selon la revendication 12, caractérisée en ce que l'on effectue la mesure à un potentiel d'environ 50 mV.

15. Méthode selon la revendication 12, caractérisée en ce que l'on effectue la mesure à un potentiel d'environ 30 mV.

16. Système d'électrodes à utiliser dans la méthode selon l'une quelconque des revendications 1 à 15 précédentes, comprenant un élément de corps d'électrode (16) ayant une portion terminale libre (21), au moins une électrode détectrice (18) s'étendant axialement à travers ladite portion terminale et ayant une superficie de la section essentiellement uniforme dans ladite portion terminale, et une électrode de référence (19) qui s'étend aussi axialement à travers la portion terminale libre (21) de l'élément de corps d'électrode, chaque électrode étant logée dans un orifice s'étendant dans le sens de la longueur à travers l'élément de corps d'électrode et ayant une partie de surface exposée au niveau de la portion terminale libre de l'élément de corps d'électrode, caractérisé en ce que l'électrode de référence est une électrode Ag/AgCl et en ce que l'électrode détectrice est une électrode conductrice de l'électricité comprenant une pâte de particules conductrices de l'électricité et d'une matière formant une pâte contenant une enzyme oxydase et un médiateur de transfert de charge, qui est réparti de façon uniforme dans la pâte et qui, sous sa forme réduite, est dissous dans la matière formant une pâte à une concentration qui est au moins suffisante pour assurer pendant la mesure une concentration essentiellement constante du médiateur de transfert de charge sous forme oxydée au niveau de la surface de l'électrode détectrice, le médiateur de transfert de charge sous forme réduite étant éventuellement présent en plus sous forme non dissoute dans la pâte.

17. Système d'électrodes selon la revendication 16, caractérisé en ce qu'il contient en outre une contre-électrode (20) qui s'étend axialement à travers la portion terminale libre (21) de l'élément de corps d'électrode (16).

18. Système d'électrodes selon la revendication 16, caractérisé en ce que la pâte contient des particules de graphite et une matière formant une pâte non polaire qui est essentiellement non miscible à l'eau et qui est capable de dissoudre le médiateur de transfert de charge sous forme réduite.

19. Système d'électrodes selon l'une quelconque des revendications 16 à 18 précédentes, caractérisé en ce que l'enzyme oxydase est la glucose oxydase qui est liée et/ou adsorbée sur les particules conductrices de l'électricité.

20. Système d'électrodes selon l'une quelconque des revendications 16 à 19 précédentes, caractérisé en ce que la concentration de la forme réduite du médiateur de transfert de charge dans la matière formant une

34

pâte est de 0,01 à 1,5 M, de préférence de 0,05 à 1,0 M, de façon davantage préférée de 0,07 à 0,7 M, et de la façon la plus préférée de 0,4 à 0,7 M.

21. Système d'électrodes selon l'une quelconque des revendications 16 à 20 précédentes, caractérisé en ce que la matière non polaire formant une pâte est de l'huile de paraffine.

22. Système d'électrodes selon l'une quelconque des revendications 16 à 21 précédentes, caractérisé en ce que l'élément de corps d'électrode est en une matière polymère.

23. Système d'électrodes selon la revendication 22, caractérisé en ce que la matière polymère est un mélange de polyéthylène haute densité et de polyéthylène basse densité, le rapport en masse des quantités de polyéthylène haute densité et basse densité étant de préférence d'environ 1.

24. Système d'électrodes selon l'une quelconque des revendications 16 à 23 précédentes, caractérisé en ce qu'il comprend deux électrodes détectrices (18), une électrode de référence Ag/AgCl (19) et une contre-électrode (20).

25. Système d'électrodes selon la revendication 24, caractérisé en ce que les deux électrodes détectrices sont du même type.

26. Système d'électrodes selon la revendication 25, caractérisé en ce que les deux électrodes détectrices sont différentes, l'une des électrodes comprenant de la glucose oxydase sous une forme catalytiquement active, l'autre électrode comprenant de la glucose oxydase sous forme inactivée.

27. Appareil de mesure pour la mise en oeuvre de la méthode selon les revendications 1 à 15 pour la mesure d'au moins un constituant d'un échantillon liquide de sang total animal ou humain, à l'aide d'une électrode selon l'une quelconque des revendications 16 à 26, caractérisé en ce qu'il comprend:

un élément de corps (10) définissant une cavité (14) ouverte vers l'extérieur destinée à recevoir le système d'électrodes (15),

un moyen d'enlèvement (27) monté sur l'élément de corps (10) de façon à pouvoir se déplacer par rapport à lui, le long d'une trajectoire qui coupe ladite cavité (14), pour enlever une section terminale externe d'une portion terminale libre du système d'électrodes (15) logé dans la cavité (14),

des moyens (30-34 et 37) pour déplacer le système d'électrodes d'un incrément vers l'extérieur chaque fois que le moyen d'enlèvement a été actionné,

un circuit de mesure électronique (35) pour le traitement des signaux de mesure reçus de l'électrode ou des électrodes du système d'électrodes, et

des moyens de raccordement pour le raccordement électrique de l'électrode ou des électrodes du système d'électrodes au circuit de mesure électronique lorsque le système d'électrodes est logé dans ladite cavité.

28. Appareil selon la revendication 27, caractérisé en ce que ledit moyen d'enlèvement définit une arête coupante (28) destinée à couper la section terminale externe de ladite portion terminale libre du système d'électrodes lors du déplacement du moyen d'enlèvement le long de ladite trajectoire.

29. Appareil selon la revendication 27 ou 28, caractérisé en ce que le moyen d'enlèvement est monté de façon à avoir un mouvement de va-et-vient le long de ladite trajectoire.

30. Appareil selon l'une quelconque des revendications 27 à 29, caractérisé en ce que le moyen d'enlèvement est monté sur l'élément de corps de l'appareil de façon à pouvoir tourner par rapport à lui.

31. Appareil selon la revendication 30, caractérisé en ce qu'il comprend en outre un moyen permettant la rotation du moyen d'enlèvement dans une seule direction.

32. Appareil selon la revendication 31, caractérisé en ce que ledit moyen permettant la rotation comprend un mécanisme à cliquet.

33. Appareil selon l'une quelconque des revendications 27 à 32, caractérisé en ce que ledit moyen mobile comprend un élément d'entraînement actionné par le mouvement dudit moyen d'enlèvement.

34. Appareil selon l'une quelconque des revendications 27 à 33, caractérisé en ce que le moyen d'enlèvement est monté sur un élément de type couvercle (11) qui peut se déplacer vers une position de fermeture non opérationnelle.

35. Appareil selon l'une quelconque des revendications 27 à 34, caractérisé en ce qu'il comprend en outre un élément de perforation de la peau (46) monté dans l'élément de corps de l'appareil (10) de façon à être mobile entre une position de retrait et une position avancée,

des moyens de décentrage (52, 53) pour amener l'élément de perforation vers sa position avancée, et

un moyen de blocage libérable (54) pour retenir l'élément de perforation dans sa position de retrait contre la charge du moyen de décentrage, grâce à quoi l'élément de perforation peut effectuer un brusque mouvement de perforation de la peau de sa position de retrait à sa position avancée lors de la libération du moyen de blocage.

36. Appareil selon l'une quelconque des revendications 16 à 35, caractérisé en ce qu'il comprend en outre une unité de visualisation (36) pour l'affichage du résultat des mesures effectuées par l'électrode ou les électrodes du système d'électrodes.

**Fig. 1**

**Fig. 2**

**Fig. 3**

**Fig. 4**

**Fig. 5**

**Fig. 6**

**Fig. 7**

**Fig. 8**

**Fig. 9**

Fig. 10

Fig. 11

**Fig. 12**     **Fig. 13**

**Fig. 14**     **Fig. 15**

41

Fig. 16    Fig. 17    Fig. 18

Fig. 19

Fig. 20

Fig. 21

EP 0 478 550 B1

Fig. 22

Fig. 23

# Fig. 24

**Fig. 25**

**Fig. 26**

EP 0 478 550 B1

Fig. 27A

FIG. 27B

FIG. 28A

# FIG. 28B

FIG. 29A

FIG. 29B

EP 0 478 550 B1

FIG. 30A

FIG. 30B

EP 0 478 550 B1

# FIG. 30C

GLUCOSE CONCENTRATION (MG/DL)

CURRENT INTEGRATION (μA.s)

□ V = 30 mV (Ag/AgCl)

+ V = 110 mV